(19) Europäisches Patentamt
European Patent Office
Office européen des brevets

(11) EP 0 534 258 B1

(12) EUROPÄISCHE PATENTSCHRIFT

(45) Veröffentlichungstag und Bekanntmachung des Hinweises auf die Patenterteilung:
**21.05.1997 Patentblatt 1997/21**

(51) Int Cl.[6]: **C07D 263/10**, C09K 19/58, C09K 19/34, C07D 413/10

(21) Anmeldenummer: **92115653.5**

(22) Anmeldetag: **14.09.1992**

(54) **Chirale Oxazoline als Dotierstoffe für flüssigkristalline Gemische**

Chiral oxazolines as dopants for liquid crystals mixtures

Oxazolines chirales comme agents dopants pour des mélanges de cristaux liquides

(84) Benannte Vertragsstaaten:
**CH DE FR GB IT LI NL**

(30) Priorität: **26.09.1991 CH 2862/91**
**07.10.1991 CH 2951/91**
**07.04.1992 CH 1128/92**

(43) Veröffentlichungstag der Anmeldung:
**31.03.1993 Patentblatt 1993/13**

(73) Patentinhaber: **F. HOFFMANN-LA ROCHE AG**
**4002 Basel (CH)**

(72) Erfinder:
- **Kelly, Stephen**
  **CH-4313 Möhlin (CH)**
- **Buchecker, Richard**
  **CH-8008 Zürich (CH)**

(74) Vertreter: **Cottong, Norbert A. et al**
**F.Hoffmann-La Roche AG**
**Patent Department (PLP),**
**124 Grenzacherstrasse**
**4070 Basel (CH)**

(56) Entgegenhaltungen:
**EP-A- 0 441 213**

- **CHEMISCHE BERICHTE Bd. 124, Nr. 5, 1991, WEINHEIM DE Seiten 1173 - 1180 C. BOLM 'Synthesis of Optically Active Bis(2-oxazolines): Crystal Structure of a 1,2- Bis (2-oxazolinyl)benzene . Zncl2 Complex'**
- **CHEMICAL ABSTRACTS, vol. 117, no. 4, 27. Juli 1992, Columbus, Ohio, US; abstract no. 367, Seite 722 ;**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue optisch aktive Dotierstoffe für Flüssigkristalle sowie flüssigkristalline Gemische, die solche Dotierstoffe enthalten und deren Verwendung für optische und elektro-optische Zwecke.

Flüssigkristallmaterialien für elektro-optische Anzeigevorrichtungen enthalten häufig einen oder mehrere optisch aktive Zusatzstoffe zur Induzierung einer chiralen Struktur. Beispielsweise wird zur Verwendung in Anzeigevorrichtungen mit verdrillt nematischer Struktur bevorzugt ein nematischer Flüssigkristall mit optisch aktivem Zusatzstoff dotiert, z.B. um eine Umkehr der Verdrillungsrichtung (reverse twist) in TN-Zellen (twisted-nematic) zu vermeiden oder um eine ausreichende Verdrillung in Zellen mit hoch verdrillter nematischer Struktur, wie STN-Zellen (super twisted-nematic), SBE-Zellen (super birefringence effect) oder OMI-Zellen (optical mode interference) zu erreichen. Ferner können cholesterische Flüssigkristalle für Phase-Change-Zellen vorzugsweise aus einem nematischen Basismaterial und einem oder mehreren optisch aktiven Dotierstoffen bestehen und ferroelektrische Flüssigkristalle für Anzeigevorrichtungen auf der Basis von chiral getilteten smektischen Phasen können vorzugsweise aus einem Material mit getilteter smektischer Phase und einem oder mehreren optisch aktiven Dotierstoffen bestehen.

Die elektro-optischen Kennlinien von Flüssigkristall-Anzeigevorrichtungen sind temperaturabhängig, was insbesondere beim Multiplexbetrieb störend ist. Es ist jedoch bekannt, dass diese Temperaturabhängigkeit mindestens teilweise kompensiert werden kann durch Zusatz von chiralem Dotierstoff, welcher eine mit steigender Temperatur abnehmende Ganghöhe induziert. Eine solche inverse Temperaturabhängigkeit wurde bisher allerdings nur für wenige Verbindungen gefunden. Sie kann aber auch durch Verwendung von mindestens 2 chiralen Dotierstoffen erreicht werden, welche eine unterschiedliche relative Temperaturabhängigkeit aufweisen und unterschiedliche Verdrillungsrichtung induzieren (US Pat. Nr. 4 264 148). Allerdings erfordert dies meist einen relativ hohen Anteil an chiralen Dotierstoffen.

Cholesterische Flüssigkristalle reflektieren Licht in einem Wellenlängenbereich, für den die Wellenlänge etwa gleich der Helixganghöhe ist. Die spektrale Breite dieses Reflexionslichtes kann durch geeignete Wahl des Flüssigkristalles variiert werden. Das reflektierte Licht ist vollständig zirkular polarisiert. Die Drehrichtung des reflektierten Lichts hängt vom Drehsinn der cholesterischen Helixstruktur ab. Das entgegengesetzt zirkular polarisierte Licht wird ungeschwächt transmittiert. Diese Eigenschaften können zur Herstellung von optischen Filtern, Polarisatoren, Analysatoren etc. ausgenutzt werden. Ferner wurden cholesterische Flüssigkristalle auch verschiedentlich für thermochrome Anwendungen und in kosmetischen Präparaten verwendet.

Cholesterische Flüssigkristalle für obige Anwendungen können vorzugsweise aus einem nematischen oder cholesterischen Basismaterial und einem oder mehreren chiralen Dotierstoffen bestehen, was eine einfache Einstellung der gewünschten Helixganghöhe erlaubt.

Um cholesterische Gemische mit einer Ganghöhe im Bereich der Wellenlänge des sichtbaren Lichts zu erreichen, sollten die chiralen Dotierstoffe ein möglichst hohes Verdrillungsvermögen aufweisen und in üblichen Flüssigkristallmaterialien gut löslich sein. Weiterhin sollten die chiralen Dotierstoffe eine ausreichende Stabilität besitzen, mit dem Mesophasetyp des Flüssigkristallmaterials gut kompatibel sein und den Mesophasenbereich nicht zu stark einschränken. Solche Eigenschaften wären auch für chirale Dotierstoffe zur Erzielung der eingangs genannten verdrillt nematischen Strukturen erwünscht, da ihr Anteil so niedrig gehalten werden könnte, dass die Eigenschaften des Flüssigkristallmaterials nur unwesentlich beeinflusst werden.

Derartige Verbindungen werden nun gemäss der vorliegenden Erfindung zur Verfügung gestellt.

Die Erfindung betrifft optisch aktive Verbindungen der allgemeinen Formel

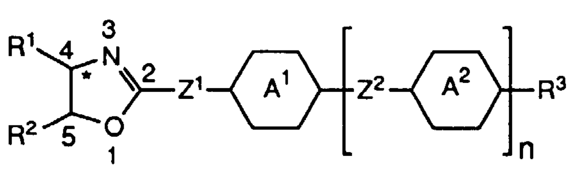

I

worin n für die Zahl 0 oder 1 steht; $R^3$ eine Gruppe $R^4$ oder eine Gruppe der allgemeinen Formeln

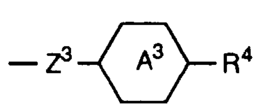

II

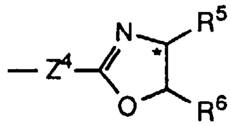

III

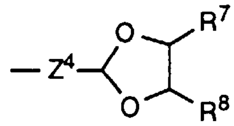

IV

oder

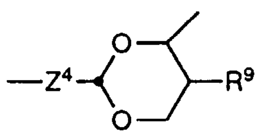

V

bezeichnet; $A^1$, $A^2$ und $A^3$ unabhängig voneinander unsubstituiertes oder einfach oder mehrfach mit Halogen, Cyano und/oder Methyl substituiertes 1,4-Phenylen, in welchem - falls unsubstituiert - gegebenenfalls 1 oder 2 CH-Gruppen durch Stickstoff ersetzt sind, trans-1,4-Cyclohexylen, trans-1,3-Dioxan-2,5-diyl, Bicyclo[2.2.2.]octan-1,4-diyl, Naphthalin-2,6-diyl, Tetralin-2,6-diyl oder trans-Decalin-2,6-diyl darstellen; $Z^1$ und $Z^4$ unabhängig voneinander eine einfache Kovalenzbindung oder $-CH_2CH_2-$ bedeuten; $Z^2$ und $Z^3$ unabhängig voneinander eine einfache Kovalenzbindung, $-CH_2CH_2-$, -COO-, -OOC-, $-CH_2O-$, $-OCH_2-$, -C≡C-, $-(CH_2)_4-$, $-(CH_2)_3O-$, $-O(CH_2)_3-$ oder die trans-Form von $-CH=CH-CH_2CH_2-$, $-CH_2CH_2-CH=CH-$, $-CH=CH-CH_2O-$ oder $-OCH_2-CH=CH-$ bezeichnen; $R^1$ und $R^5$ unabhängig voneinander Alkyl mit 1 bis 12 Kohlenstoffatomen, Alkoxymethyl mit 2 bis 12 Kohlenstoffatomen, p-Alkoxyphenyl oder p-Alkoxybenzyl bedeuten; $R^2$ und $R^6$ unabhängig voneinander Wasserstoff, Alkyl mit 1 bis 12 Kohlenstoffatomen oder Phenyl bedeuten; $R^7$ und $R^8$ unabhängig voneinander Wasserstoff, Alkyl mit 1 bis 12 Kohlenstoffatomen, Phenyl oder Alkoxycarbonyl mit 2 bis 12 Kohlenstoffatomen bedeuten, mit der Massgabe, dass nicht $R^7$ und $R^8$ gleichzeitig Wasserstoff darstellen; $R^9$ unsubstituiertes oder einfach oder mehrfach mit Fluor substituiertes Alkyl oder Alkenyl mit 1 bzw. 2 bis 9 Kohlenstoffatomen, in welchen eine $-CH_2$-Gruppe durch -O- ersetzt sein kann, bedeutet; $R^4$ Wasserstoff, Halogen, Cyano, Trifluormethyl, Trifluormethoxy oder unsubstituiertes oder einfach oder mehrfach mit Halogen, Cyano und/oder Methyl substituiertes Alkyl oder Alkenyl mit 1 bzw. 2 bis 12 Kohlenstoffatomen bezeichnet, in welchen eine oder zwei nicht benachbarte Methylengruppen durch -O-, -COO- und/oder -OOC- ersetzt sein können; und der in Formel I dargestellte Oxazolin-Ring in optisch aktiver Form vorliegt, mit der Massgabe, dass nicht gleichzeitig $R^3$ eine Gruppe der Formel III und n 0 bedeuten.

Die Verbindungen der Formel I sind sehr gut löslich in üblichen Flüssigkristallmaterialien und ermöglichen eine sehr hohe Verdrillung der Flüssigkristallstruktur. Im Unterschied zu bekannten Materialien mit hohen Verdrillungsvermögen werden zudem die Klärpunkte von Flüssigkristallen bei Zusatz von Verbindungen der Formel I in der Regel nicht oder nur unwesentlich gesenkt. Viele der erfindungsgemässen Verbindungen besitzen sogar selbst flüssigkristalline Eigenschaften. Die Verbindungen der Formel I sind leicht herstellbar, relativ niederviskos und ausreichend stabil gegen elektrische und magnetische Felder. Sie erfüllen daher in optimaler Weise die oben genannten Anforderungen.

Die Eigenschaften der Verbindungen der Formel I können je nach Zahl und Bedeutung der Ringe und der Substituenten in breiten Bereichen variiert werden. Beispielsweise führen aromatische Ringe zu höheren und gesättigte Ringe zu tieferen Werten der optischen Anisotropie. Eine Erhöhung des Klärpunktes kann beispielsweise durch Einführung eines weiteren Ringes erreicht werden. Dementsprechend besitzen Verbindungen der Formel I, worin n die Zahl 1 und/oder $R^3$ eine Gruppe der Formel II bedeutet, oft selbst flüssigkristalline Eigenschaften. Polare Endgruppen, wie Cyano, Halogen, Trifluormethyl oder Trifluormethoxy, und Ringe, wie Pyrimidin-2,5-diyl oder trans-1,3-Dioxan-2,5-diyl, erhöhen die dielektrische Anisotropie; Ringe, wie Pyridazin-3,6-diyl oder 2,3-Dicyano-1,4-phenylen, vermindern die dielektrische Anisotropie. Laterale Halogen- oder Cyanosubstituenten ergeben einen Beitrag zur Dielektrizitätskonstante sowohl parallel als auch senkrecht zur Molekküllängsachse, was je nach Substitutionsmuster zur Erhöhung oder Verminderung der dielektrischen Anisotropie ausgenützt werden kann. Ferner kann durch laterale Substituenten an einem oder mehreren Ringen eine allfällige Tendenz zur Ausbildung hochgeordneter smektischer Phasen oft weitgehend unterdrückt und oft auch die Löslichkeit verbessert werden. Weiterhin können durch eine C=C-Doppelbindung in der Seitenkette die elastischen Eigenschaften, die Schwellenspannungen, die Ansprechzeiten, die Mesophasen etc. weiter modifiziert werden.

Die erfindungsgemässen Verbindungen ermöglichen daher neben der Induzierung einer hohen Verdrillung zusätzlich die Optimierung flüssigkristalliner und elektro-optischer Eigenschaften in einem weiten Bereich je nach Anwendung und gewünschten Eigenschaften.

Der Ausdruck "Halogen" bezeichnet im Rahmen der vorliegenden Erfindung Fluor, Chlor, Brom oder Jod, insbesondere Fluor oder Chlor.

Der Ausdruck "unsubstituiertes oder einfach oder mehrfach mit Halogen, Cyano und/oder Methyl substituiertes 1,4-Phenylen, in welchem - falls unsubstituiert - gegebenenfalls 1 oder 2 CH-Gruppen durch Stickstoff ersetzt sind," umfasst Gruppen wie 1,4-Phenylen, 2-Fluor-1,4-phenylen, 2,3-Difluor-1,4-phenylen, 2-Chlor-1,4-phenylen, 2-Brom-1,4-phenylen, 2-Cyano-1,4-phenylen, 2,3-Dicyano-1,4-phenylen, 2-Methyl-1,4-phenylen, Pyridin-2,5-diyl, Pyrazin-2,5-diyl, Pyrimidin-2,5-diyl, Pyridazin-3,6-diyl und dergleichen. Bevorzugte Gruppen sind 1,4-Phenylen, 2-Fluor-1,4-phenylen, 2,3-Difluor-1,4-phenylen, Pyridin-2,5-diyl, Pyrazin-2,5-diyl und Pyrimidin-2,5-diyl.

Der Ausdruck "Tetralin-2,6-diyl" bezeichnet 1,2,3,4-Tetrahydronaphthalin-2,6-diyl. Der Ausdruck "trans-Decalin-2,6-diyl" umfasst von trans-Decahydronaphthalin abgeleitete, 2,6-disubstituierte Gruppen, insbesondere (4a$\alpha$H,7a$\beta$H)-Decahydronaphthalin-2$\alpha$,6$\beta$-diyl.

Der Ausdruck "Alkyl" umfasst geradkettige und verzweigte, gegebenenfalls chirale Gruppen mit 1-12 Kohlenstoffatomen, wie Methyl, Aethyl, Propyl, Isopropyl, Butyl, Isobutyl, tert.Butyl, 2-Butyl (=1-Methylpropyl), 2-Methylbutyl, Pentyl, Hexyl, Heptyl, Octyl, 2-Octyl (=1-Methylheptyl), Nonyl, Decyl, Undecyl, Dodecyl und dergleichen. Bevorzugt sind Alkylreste mit 1 bis 7 Kohlenstoffatomen, insbesondere Alkylreste mit 1 bis 5 Kohlenstoffatomen, wie beispielsweise Methyl, Isopropyl und tert.Butyl.

Der Ausdruck "Alkoxymethyl" umfasst geradkettige und verzweigte, gegebenenfalls chirale Gruppen mit 2 bis 12, vorzugsweise 2 bis 7 Kohlenstoffatomen, wie beispielsweise Methoxymethyl, Ethoxymethyl, Propyloxymethyl, Butyloxymethyl, Isopropyloxymethyl, tert.-Butyloxymethyl und dergleichen; ganz besonders bevorzugt sind solche Reste mit 2 bis 5 stoffatomen.

Der Ausdruck "Alkoxycarbonyl" umfasst geradkettige und verzweigte, gegebenenfalls chirale Gruppen mit 2-12 Kohlenstoffatomen, wie Methoxycarbonyl, Aethoxycarbonyl, Propyloxycarbonyl, Isopropyloxycarbonyl, Butyloxycarbonyl, Isobutyloxycarbonyl, (2-Butyl)oxycarbonyl, (2-Methylbutyl)oxycarbonyl, Pentyloxycarbonyl, Hexyloxycarbonyl und dergleichen. Bevorzugt sind Gruppen mit 2 bis 7 Kohlenstoffatomen.

In den Ausdrücken "p-Alkoxyphenyl" und "p-Alkoxybenzyl" bedeutet Alkoxy einen geradkettigen oder einen verzweigten, gegebenenfalls chiralen Rest mit 1 bis 7 Kohlenstoffatomen. Beispiele bevorzugter p-Alkoxyphenyl-bzw. p-Alkoxybenzyl-Gruppen sind 4-Methoxyphenyl, 4-Aethoxyphenyl, 4-Propyloxyphenyl, 4-Butyloxyphenyl, 4-Pentyloxyphenyl, 4-Hexyloxyphenyl, 4-Heptyloxyphenyl, 4-Methoxybenzyl, 4-Aethoxybenzyl, 4-Propyloxybenzyl, 4-Butyloxybenzyl, 4-Pentyloxybenzyl, 4-Hexyloxybenzyl, 4-Heptyloxybenzyl und dergleichen.

Der Ausdruck "unsubstituiertes oder einfach oder mehrfach mit Fluor substituiertes Alkyl oder Alkenyl mit 1 bzw. 2 bis 9 Kohlenstoffatomen, in welchem eine -$CH_2$-Gruppen durch -O- ersetzt sein kann" bedeutet im Rahmen der vorliegenden Erfindung geradkettige und verzweigte (gegebenenfalls chirale) Reste mit 1 bzw. 2 bis 9 Kohlenstoffatomen wie Alkyl, 3E-Alkenyl, 4-Alkenyl, Alkenyl mit endständiger Doppelbindung, Alkoxy, 2E-Alkenyloxy, 3-Alkenyloxy, Alkenyloxy mit endständiger Doppelbindung, Alkoxyalkyl, Alkenyloxyalkyl, Alkoxyalkenyl, Fluoroalkyl und dergleichen. Bevorzugt werden Reste mit 1 bzw. 2 bis 7 Kohlenstoffatomen. Beispiele bevorzugter Reste sind Methyl, Aethyl, Propyl, Butyl, Pentyl, Hexyl, Heptyl, 1-Methylpropyl, 2-Methylbutyl, 3-Methylpentyl, 3-Butenyl, 3E-Pentenyl, 3E-Hexenyl, 3E-Heptenyl, 4-Pentenyl, 4Z-Hexenyl, 4Z-Heptenyl, 5-Hexenyl, 6-Heptenyl, Methoxy, Aethoxy, Propyloxy, Butyloxy, Pentyloxy, Hexyloxy, Heptyloxy, 1-Methylpropyloxy, 2-Methylbutyloxy, 3-Methylpentyloxy, Allyloxy, 2E-Butenyloxy, 2E-Pentenyloxy, 2E-Hexenyloxy, 2E-Heptenyloxy, 3-Butenyloxy, 3Z-Pentenyloxy, 3Z-Hexenyloxy, 3Z-Heptenyloxy, 4-Pentenyloxy, 5-Hexenyloxy, 6-Heptenyloxy, Methoxymethyl, Aethoxymethyl, Propyloxymethyl, Allyloxymethyl, 2-Fluoräthyl, 3-Fluorpropyl, 4-Fluorbutyl, 5-Fluorpentyl, 6-Fluorhexyl, 1-Fluorpropyl, 1-Fluorpentyl, 2-Fluorpropyloxy, 2-Fluorbutyloxy, 2-Fluorpentyloxy, 2-Fluorhexyloxy, 2-Fluorbutyloxy und dergleichen. Ganz besonders bevorzugt haben die obengenannten Reste 1 bzw. 2 bis 5 Kohlenstoffatome.

Der Ausdruck "unsubstituiertes oder einfach oder mehrfach mit Halogen, Cyano und/oder Methyl substituiertes Alkyl oder Alkenyl, in welchen eine oder zwei nicht benachbarte Methylengruppen durch -O-, -COO- und/oder -OOC- ersetzt sein können" umfasst geradkettige und verzweigte (gegebenenfalls chirale) Reste mit 1 bzw. 2 bis 12 Kohlenstoffatomen wie Alkyl, 1E-Alkenyl, 3E-Alkenyl, 4-Alkenyl, Alkenyl mit endständiger Doppelbindung, Alkoxy, 2E-Alkenyloxy, 3-Alkenyloxy, Alkenyloxy mit endständiger Doppelbindung, Alkoxyalkyl, Alkenyloxyalkyl, Alkoxycarbonyl, Alkoxycarbonylalkoxy (z.B. 1-(Alkoxycarbonyl)-1-äthoxy), Alkoxycarbonylalkoxycarbonyl (z.B. (1-Alkoxycarbonyl)-1-äthoxycarbonyl), Alkanoyloxy, 1-Haloalkyl, 2-Haloalkyl, 2-Haloalkoxy, 2-Haloalkoxycarbonyl, 1-Cyanoalkyl, 2-Cyanoalkyl, 2-Cyanoalkoxy, 2-Cyanoalkoxycarbonyl, 1-Methylalkyl, 2-Methylalkyl, 1-Methylalkoxy, 2-Methylalkoxy, 2-Methylalkoxycarbonyl und dergleichen. Beispiele bevorzugter Reste sind Methyl, Aethyl, Propyl, Butyl, Pentyl, Hexyl, Heptyl, Octyl, Nonyl, Decyl, Undecyl, Dodecyl, 1-Methylpropyl, 1-Methylheptyl, 2-Methylbutyl, 3-Methylpentyl, Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl,

4-Pentenyl, 4Z-Hexenyl, 4Z-Heptenyl, 5-Hexenyl, 6-Heptenyl, 7-Octenyl, 8-Nonenyl, 9-Decenyl, 10-Undecenyl, 11-Dodecenyl, Methoxy, Aethoxy, Propyloxy, Butyloxy, Pentyloxy, Hexyloxy, Heptyloxy, Octyloxy, Nonyloxy, Decyloxy, Undecyloxy, Dodecyloxy, 1-Methylpropyloxy, 1-Methylheptyloxy, 2-Methylbutyloxy, 3-Methylpentyloxy, Allyloxy, 2E-Butenyloxy, 2E-Pentenyloxy, 2E-Hexenyloxy, 2E-Heptenyloxy, 3-Butenyloxy, 3Z-Pentenyloxy, 3Z-Hexenyloxy, 3Z-Heptenyloxy, 4-Pentenyloxy, 5-Hexenyloxy, 6-Heptenyloxy, 7-Octenyloxy, 8-Nonenyloxy, 9-Decenyloxy, 10-Undecenyloxy, 11-Dodecenyloxy, Methoxymethyl, Aethoxymethyl, Propyloxymethyl, Allyloxymethyl, Methoxycarbonyl, Aethoxycarbonyl, Propyloxycarbonyl, 1-Methylpropyloxycarbonyl, 1-(Methoxycarbonyl)äthoxy, 1-(Aethoxycarbonyl)äthoxy, 1-(Methoxycarbonyl)äthoxycarbonyl, 1-(Aethoxycarbonyl)äthoxycarbonyl, 1-(Isopropyloxycarbonyl)äthoxycarbonyl, 1-(Butyloxycarbonyl)äthoxycarbonyl, Acetoxy, Propionyloxy, Butyryloxy, 1-Fluorpropyl, 1-Fluorpentyl, 1-Chlorpropyl, 2-Fluorpropyl, 2-Fluorpentyl, 2-Chlorpropyl, 2-Fluorpropyloxy, 2-Fluorbutyloxy, 2-Fluorpentyloxy, 2-Fluorhexyloxy, 2-Chlorpropyloxy, 2-Fluorbutyloxy, 2-Fluorpropyloxycarbonyl, 2-Fluorbutyloxycarbonyl, 2-Fluorpentyloxycarbonyl, 2-Fluor-3-methylbutyloxycarbonyl, 2-Fluor-4-methylpentyloxycarbonyl, 2-Chlorpropyloxycarbonyl, 1-Cyanopropyl, 1-Cyanopentyl, 2-Cyanopropyl, 2-Cyanopentyl, 2-Cyanopropyloxy, 2-Cyanobutyloxy, 2-Cyanopentyloxy, 2-Cyanohexyloxy, 2-Cyanopropyloxycarbonyl, 2-Cyanobutyloxycarbonyl, 2-Cyano-3-methylbutyloxycarbonyl, 2-Cyano-4-methylpentyloxycarbonyl und dergleichen. Vorzugsweise besitzen die obengenannten Reste 1 bzw. 2 bis 12 Kohlenstoffatome; ganz besonders bevorzugt sind jedoch die Reste $R^4$ mit 1 bzw. 2 bis 7 Kohlenstoffatomen.

Formel I umfasst optisch aktive Verbindungen der folgenden allgemeinen Formeln

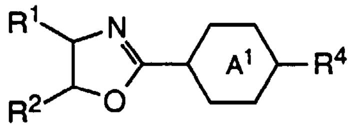

I-1

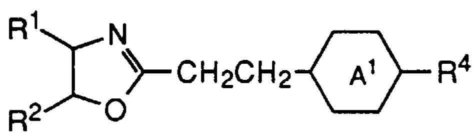

I-2

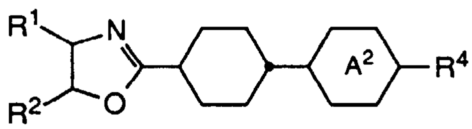

I-3

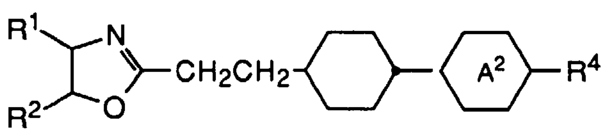

I-4

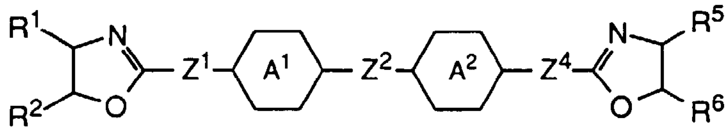

I-6

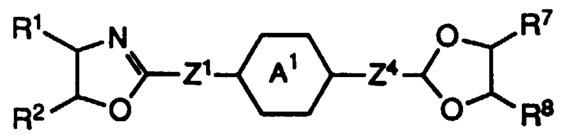

I-7

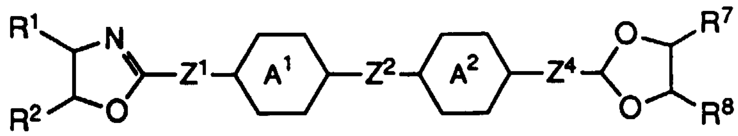

I-8

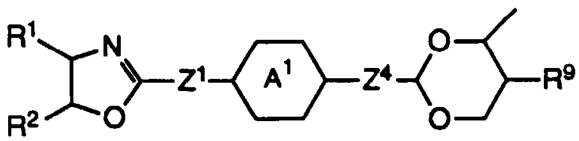

I-9

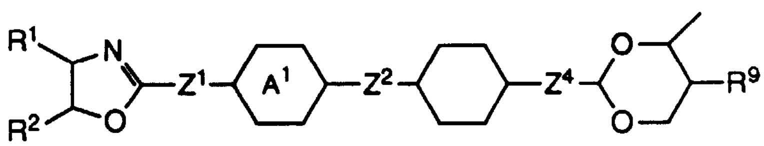

I-10

worin $R^1$, $R^2$, $R^4$, $R^5$, $R^6$, $R^7$, $R^8$, $R^9$, $A^1$, $A^2$, $Z^1$, $Z^2$,und $Z^4$ jeweils die obigen Bedeutungen haben.

Die Verbindungen der Formel I besitzen mindestens einen optisch aktiven Oxazolin-Ring. Der in Formel I dargestellte Oxazolin-Ring besitzt in Position 4 ein chirales Kohlenstoffatom und, falls $R^2$ von Wasserstoff verschieden ist, in Position 5 ein weiteres chirales Kohlenstoffatom. Es ist dem Fachmann klar, dass zur Erzielung optischer Aktivität das Kohlenstoffatom in Position 4 des Oxazolin-Ringes ganz oder überwiegend in R- oder S-Form vorliegen muss und, falls $R^1$ und $R^2$ identisch sind, das Kohlenstoffatom in Position 5 des Oxazolin-Ringes ganz oder überwiegend die gleiche Konfiguration aufweisen sollte wie das Kohlenstoffatom in Position 4, oder grundsätzlich auch ein R/S-Verhältnis von 50:50 aufweisen kann. Um ein möglichst hohes Verdrillungsvermögen zu erzielen, sollte das Kohlenstoffatom in Position 4 des Oxazolin-Ringes vorzugsweise in optisch möglichst reiner Form in R- oder S-Konfiguration vorliegen und, falls $R^2$ von Wasserstoff verschieden ist, das Kohlenstoffatom in Position 5 des Oxazolin-Ringes vorzugsweise in optisch möglichst reiner Form in jener Konfiguration vorliegen, die das Verdrillungsvermögen verstärkt. Bevorzugte optische Isomere mit hohen Verdrillungsvermögen sind die (4R)-Isomere und die (4S)-Isomere der Verbindungen der Formel I, worin $R^2$ Wasserstoff bedeutet, und die (4R,5R)-Isomere und die (4S,5S)-Isomere der Verbindungen der Formel I, worin $R^2$ Alkyl oder Phenyl bedeutet.

Besonders bevorzugt sind im allgemeinen diejenigen Verbindungen der Formeln I bzw. I-1 bis I-10, worin $A^1$, $A^2$ und $A^3$ unabhängig voneinander unsubstituiertes oder einfach oder mehrfach mit Halogen, Cyano und/oder Methyl substituiertes 1,4-Phenylen (insbesonderes 1,4-Phenylen, 2-Fluor-1,4-phenylen oder 2,3-Difluor-1,4-phenylen) oder trans1,4-Cyclohexylen darstellen oder eine der Gruppen $A^1$, $A^2$ und $A^3$ auch Pyrimidin-2,5-diyl oder Pyridin-2,5-diyl darstellt, und ferner eine der gegebenenfalls vorhandenen Gruppen $Z^2$ und $Z^3$ eine einfache Kovalenzbindung, -$CH_2CH_2$-, -COO- oder -OOC- (insbesondere eine einfache Kovalenzbindung) bezeichnet und, falls vorhanden, die andere der Gruppen $Z^2$ und $Z^3$ eine einfache Kovalenzbindung bezeichnet. Diese Verbindungen sind sehr stabil und in der Regel besonders leicht zugänglich aus bekannten Zwischenprodukten von Flüssigkristallen.

Das Verdrillungsvermögen der erfindungsgemässen Verbindungen wird hauptsächlich durch den optisch aktiven Oxazolin-Rest bestimmt ; es ist daher klar, dass die eben genannten bevorzugten Ringe und Brückengruppen lediglich die flüssigkristallinen Eigenschaften der Verbindungen beeinflussen, und dass daher auch eine oder mehrere dieser besonders bevorzugten Ringe und Brückengruppen durch die weiteren in Formel I für $A^1$, $A^2$ und $A^3$ genannten Ringe und für $Z^3$ und $Z^4$ genannten Brückengruppen ersetzt werden können, um einen ähnlichen Effekt und ebenfalls gute Kompatibilität mit üblichen Flüssigkristallen zu erhalten.

$Z^1$ und $Z^2$ in obigen Formeln I bzw. I-1 bis I-10 können gleiche oder verschiedene Bedeutungen haben. Vorzugs-

weise steht jedoch $Z^1$ und/oder $Z^2$ für eine einfache Kovalenzbindung.

Beispiele besonders bevorzugter Verbindungen der Formel I sind die optisch aktiven Verbindungen der folgenden allgemeinen Formeln

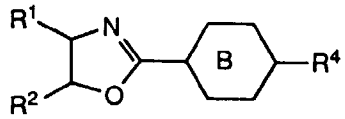

I-11

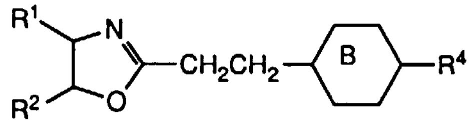

I-12

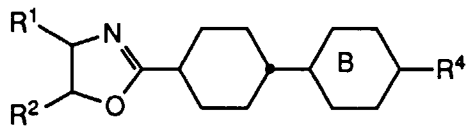

I-13

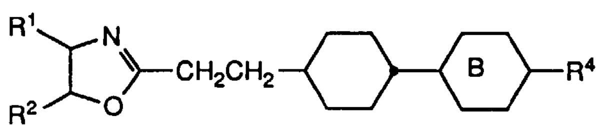

I-14

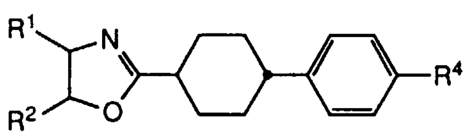

I-15

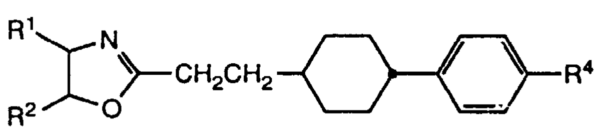

I-16

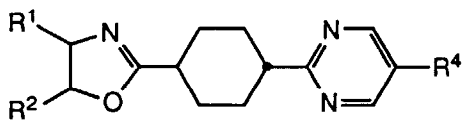

I-17

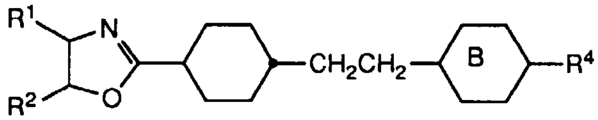

I-18

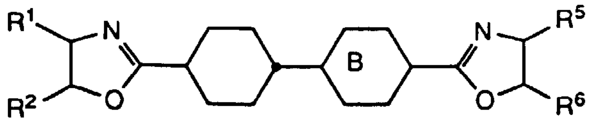

I-21

worin $R^1$, $R^2$, $R^4$, $R^5$ und $R^6$ die obigen Bedeutungen haben, und Ring B unsubstituiertes oder einfach oder mehrfach mit Halogen substituiertes 1,4-Phenylen (vorzugsweise 1,4-Phenylen, 2-Fluor-1,4-phenylen oder 2,3-Difluor-1,4-phenylen) oder trans-1,4-Cyclohexylen darstellt.

In den obigen Formeln I, I-1 bis I-4 und I-11 bis I-18 kann $R^4$ jeweils vorzugsweise Halogen (insbesondere Fluor oder Chlor), Cyano, Trifluormethyl, Trifluormethoxy, Alkyl, Alkenyl, Alkoxy oder Alkenyloxy bezeichnen. In einem Alkylrest $R^4$ können jedoch gewünschtenfalls auch eine oder zwei nicht benachbarte Methylengruppen durch -O-, -COO- und/oder -OOC- ersetzt und/oder eine Methylengruppe durch -CHX- (worin X Halogen, Cyano oder Methyl bedeutet) ersetzt werden. Diese Möglichkeit lässt sich z.B. ausnutzen zur Erhöhung des Verdrillungsvermögens durch Einführung chiraler Gruppen ausgehend von einfachen, optisch aktiven Verbindungen. Bevorzugte Beispiele derartiger Gruppen sind die von optisch aktiver Milchsäure abgeleiteten Reste, wie 1-(Alkoxycarbonyl)äthoxy und 1-(Alkoxycarbonyl)äthoxycarbonyl. $R^4$ in den obigen Formeln kann daher vorzugsweise jeweils auch einen dieser Reste bedeuten.

$R^4$ steht in der Regel für Alkyl oder Alkenyl mit 1 bzw. 2 bis 12 Kohlenstoffatomen. Besonders bevorzugte Reste $R^4$ sind diejenigen mit 1 bzw. 2 bis 7 Kohlenstoffatomen, insbesondere jedoch diejenigen mit 1 bzw. 2 bis 5 Kohlenstoffatomen.

Die Verbindungen der Formel I können ferner eine Endgruppe der Formel III, d.h. einen weiteren Oxazolin-Rest aufweisen. Diese Gruppe kann grundsätzlich optisch inaktiv sein. Bevorzugt sind jedoch Gruppen der Formel III, welche in einer optisch aktiven Form vorliegen, die das Verdrillungsvermögen verstärkt. Solche, auch besonders leicht herstellbare Verbindungen mit erhöhtem Verdrillungsvermögen sind diejenigen, worin beide Oxazolin-Reste die gleiche Konfiguration und die gleiche Reste aufweisen. Vorzugsweise kann daher in Formel III $R^5$ die gleiche Bedeutung und am benachbarten C-Atom die gleiche Konfiguration wie $R^1$ besitzen und $R^6$ die gleiche Bedeutung und am benachbarten C-Atom die gleiche Konfiguration wie $R^2$ besitzen. Dies gilt sinngemäss auch für die beiden Oxazolin-Reste in den Formeln I-5, I-6, I-19, I-20, I-21 und I-22.

Von den Verbindungen der Formel I, worin $R^3$ eine Gruppe der Formel IV oder der Formel V bedeutet - d.h. I-7, I-8, I-9 und I-10 - sind diejenigen bevorzugt, in welchen der Dioxolanring (Formel IV) bzw. der Dioxanring (Formel V) in optisch aktiver Form vorliegt.

$R^7$ und $R^8$ besitzen - sofern verschieden von Wasserstoff oder Phenyl - in der Regel 1 bis 12 Kohlenstoffatome. Im allgemeinen werden Reste $R^7$ und $R^8$ mit 1 bis 7 Kohlenstoffatomen, insbesondere jedoch diejenigen mit 1 bis 5 Kohlenstoffatomen bevorzugt.

Der Rest $R^9$ besitzt in der Regel 1 bis 9 Kohlenstoffatome. Besonders bevorzugte Reste $R^9$ besitzen 1 bis 5 Kohlenstoffatome.

Bevorzugte optische Isomere sind somit die Verbindungen mit der in der folgenden allgemeinen Formel angegebenen relativen Konfiguration:

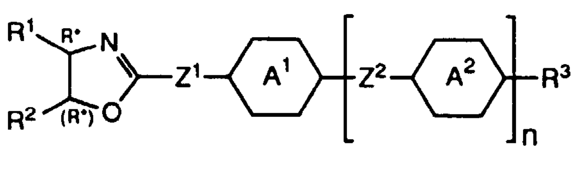

IA

worin $R^3$ eine Gruppe $R^4$ oder eine Gruppe der allgemeinen Formel

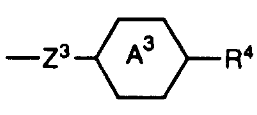

II

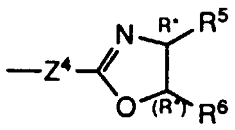

IIIA

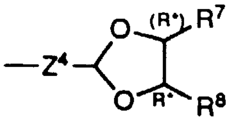

IVA

oder

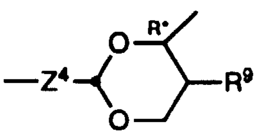

VA

bezeichnet; n, $A^1$, $A^2$, $A^3$, $Z^1$, $Z^2$, $Z^3$, $Z^4$, $R^1$, $R^2$, $R^4$, $R^5$, $R^6$, $R^7$, $R^8$ und $R^9$ die obigen Bedeutungen haben; das Chiralitätszentrum (R*) fehlt, wenn $R^2$ Wasserstoff bedeutet, und steht für R*, wenn $R^2$ von Wasserstoff verschieden ist; und die mit R* bezeichneten, asymmetrischen Kohlenstoffatomen alle in der R-Konfiguration oder alle in der S-Konfiguration vorliegen.

Formel IA umfasst die mit R* angegebenen optischen Isomere sowie deren optische Antipoden (S-Form), welche das gleiche Verdrillungsvermögen aber mit entgegengesetzter Verdrillungsrichtung besitzen.

Bevorzugte, optisch aktive Verbindungen der Formel IA sind diejenigen, worin $R^1$ und $R^2$ verschiedene oder vorzugsweise gleiche Bedeutung haben. Ganz besonders bevorzugt sind die Verbindungen, worin $R^1$ $C_1$-$C_5$-Alkyl, p-Alkoxyphenyl oder p-Alkoxybenzyl, insbesondere jedoch Isopropyl und tert.Butyl bezeichnet. Diese Verbindungen sind leicht zugänglich aus den entsprechenden, optisch aktiven 2-Amino-1-äthylalkoholen. Ganz besonders bevorzugt sind demnach herstellungsmässig diejenigen Verbindungen der Formeln I-1 bis I-21, worin jeweils $R^1$ Isopropyl oder tert. Butyl und $R^2$ Wasserstoff bezeichnen.

In obiger Formel IA und in den oben als bevorzugt genannten Untergruppen von Verbindungen der Formel I können die Ringe $A^1$ ,$A^2$ und $A^3$, Ring B, die Brückengruppen $Z^1$, $Z^2$, $Z^3$, $Z^4$ insbesondere jeweils die im Zusammenhang mit den Formeln I und I-1 bis I-21 angegebenen, bevorzugten Bedeutungen haben.

Die erfindungsgemässen Verbindungen können leicht in an sich bekannter Weise hergestellt werden. Sie können beispielsweise dadurch hergestellt werden, dass man ein Säurechlorid der Formel $Cl{\cdot}OC\text{-}Z^1\text{-}A^1\text{-}(Z^2\text{-}A^2)_n\text{-}R^3$ mit einem optisch aktiven Aminoalkohol der Formel $R^1\text{-}CH(NH_2)\text{-}CH(OH)\text{-}R^2$ umsetzt. Die Umsetzung des Säurechlorids mit dem Aminoalkohol kann in an sich bekannter Weise erfolgen. Zweckmässigerweise erfolgt die Umsetzung in einem inerten organischen Lösungsmittel (beispielsweise Dichlormethan). Temperatur und Druck sind nicht kritisch, vorzugsweise wird jedoch bei 0°C und Atmosphärendruck gearbeitet. Die Bildung des Oxazolin-Ringes kann in an sich bekannter Weise erfolgen. Zweckmässigerweise erfolgt die Umsetzung in einem inerten Lösungsmittel (beispielsweise Dichlormethan) in Gegenwart von Thionylchlorid.

Ein gegebenenfalls vorhandener, zweiter Oxazolin-Ring (wenn $R^3$ eine Gruppe der Formel III bedeutet) kann in analoger Weise ebenfalls aus Säurechlorid und Aminoalkohol gebildet werden. Sind beide Aminoalkohole [$R^1$-CH($NH_2$)-CH(OH)-$R^2$ und $R^5$-CH($NH_2$)-CH(OH)-$R^6$] identisch, können gewünschtenfalls beide Oxazolin-Ringe in einer Stufe gebildet werden.

Enthält die Verbindung der Formel I eine oder mehrere Estergruppen in $Z^2$, $Z^3$ und/oder $R^4$, so kann vorzugsweise die betreffende Veresterung auch erst nach der Bildung des Oxazolin-Ringes erfolgen. Gewünschtenfalls können auch Aethergruppen und andere funktionelle Gruppen erst nach der Bildung des Oxazolin-Ringes eingeführt werden.

Derartige Methoden sind dem Fachmann grundsätzlich bekannt, z.B. aus der Herstellung flüssigkristalliner Dioxane. Die zur Herstellung benötigten Aminoalkohole sind bekannte oder Analoge bekannter Verbindungen. Die als Ausgangsmaterialien benötigten Säurechloride sind ebenfalls bekannte Verbindungen oder können nach an sich bekannten Methoden hergestellt werden. Derartige Verbindungen und Methoden sind z.B. bekannt aus US Pat. Nr. 4 676 604, US Pat. Nr. 4 621 901 und EP-A-0168683.

Die Erfindung betrifft ebenfalls flüssigkristalline Gemische enthaltend ein flüssigkristallines Trägermaterial und eine oder mehrere optisch aktive Verbindungen der Formel I. Als Trägermaterial eignen sich grundsätzlich alle Flüssigkristallmaterialien, welche eine verdrillbare Flüssigkristallphase mit einem adäquaten Mesophasenbereich aufweisen. Besonders geeignet sind die Verbindungen der Formel I als chirale Dotierstoffe für nematische oder cholesterische Trägermaterialien. Das flüssigkristalline Trägermaterial kann eine Einzelverbindung oder ein Gemisch sein und besitzt vorzugsweise einen Klärpunkt von mindestens etwa 60°C.

Der Anteil an chiralem Dotierstoff der Formel I ist im wesentlichen durch dessen Verdrillungsvermögen und die gewünschte Ganghöhe bestimmt. Der Anteil an chiralem Dotierstoff kann daher je nach Anwendung in einem breiten Bereich variieren und beispielsweise etwa 0,1-30 Gew.% betragen. Für Anzeigevorrichtungen auf der Basis von Flüssigkristallen mit verdrillt nematischer Struktur ist je nach Zellentyp und -dicke meist eine Ganghöhe von etwa 3-40 µm erforderlich und daher ein entsprechend kleiner Anteil ausreichend. Demgegenüber werden für Anwendungen, welche auf der Reflexion von sichtbarem Licht durch cholesterische Schichten beruhen, Ganghöhen von weniger als 2 µm, beispielsweise etwa 0,4-0,6 µm benötigt, was einen entsprechend höheren Anteil an chiralem Dotierstoff erfordert.

Geeignete flüssigkristalline Trägermaterialien sind in grosser Zahl bekannt und im Handel erhältlich. In der Regel sind flüssigkristalline Gemische enthaltend 2 oder mehrere Komponenten als Trägermaterialien bevorzugt. Grundsätzlich kann aber auch eine flüssigkristalline Verbindung als Trägermaterial verwendet werden, wenn sie eine ausreichend breite Mesophase aufweist.

Als Komponenten für flüssigkristalline Trägermaterialien eignen sich insbesondere Verbindungen der folgenden allgemeinen Formeln

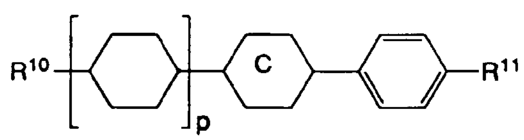

VI

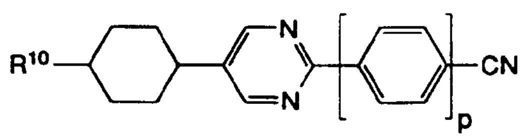

VII

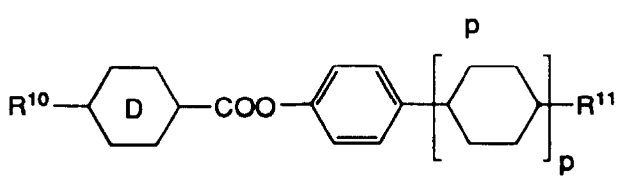

VIII

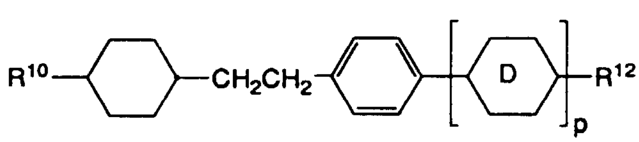

IX

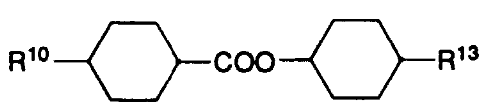

X

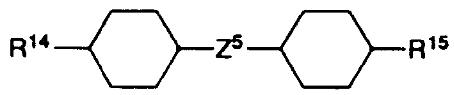

XI

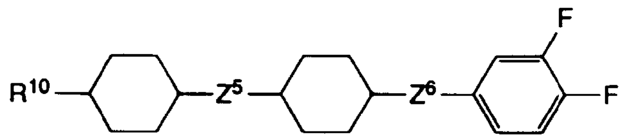

XII

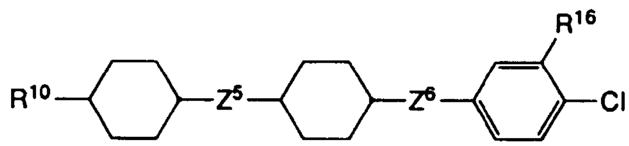

XIII

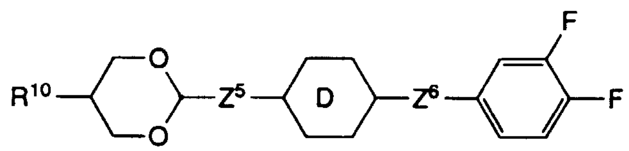

XIV

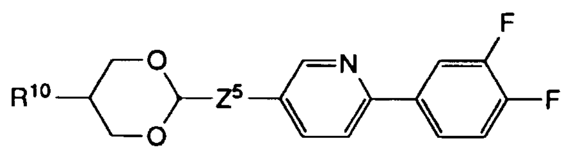

XV

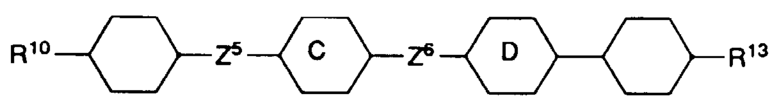

XVI

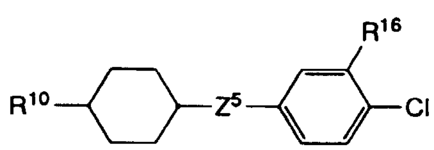

XVII

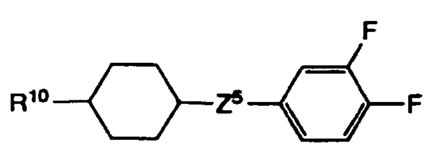

XVIII

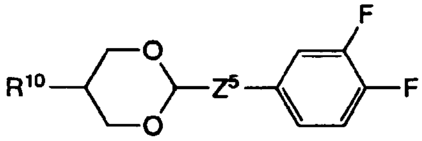

XIX

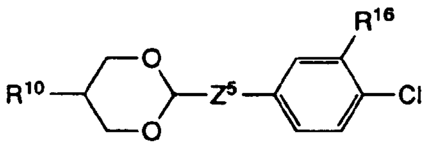

XX

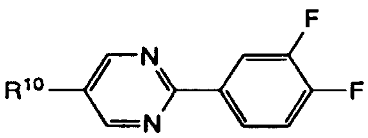

XXI

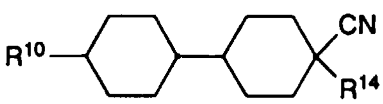

XXII

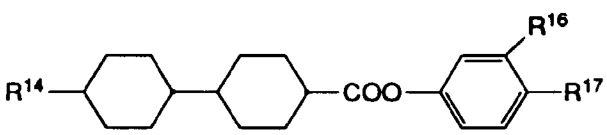

XXIII

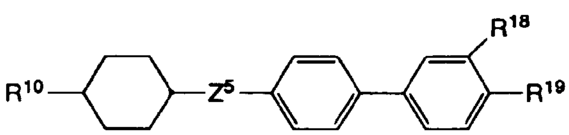

XXIV

worin $R^{10}$ und $R^{13}$ Alkyl, Alkoxyalkyl, 3E-Alkenyl, 4-Alkenyl oder an gesättigten Ringen auch 1E-Alkenyl bedeutet; p 0 oder 1 bedeutet; Ring C 1,4-Phenylen, Pyridin-2,5-diyl, Pyrimidin-2,5-diyl, trans-1,4-Cyclohexylen oder trans-1,3-Dioxan-2,5-diyl bezeichnet; $R^{11}$ Cyano, Isothiocyanato, Fluor, Alkyl, 3E-Alkenyl, 4-Alkenyl, Alkoxy, 2E-Alkenyloxy, 3-Alkenyloxy oder 1-Alkinyl darstellt; Ring D 1,4-Phenylen oder trans1,4-Cyclohexylen bedeutet; $R^{12}$ Alkyl, 3E-Alkenyl, 4-Alkenyl, oder an trans-1,4-Cyclohexylen auch 1E-Alkenyl, oder an 1,4-Phenylen auch Cyano, Isothiocyanato, Alkoxy, 2E-Alkenyloxy oder 3-Alkenyloxy bezeichnet; $R^{14}$ Alkyl, 1E-Alkenyl, 3E-Alkenyl oder 4-Alkenyl bedeutet; $R^{15}$ Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl darstellt; $Z^5$ und $Z^6$ eine einfache Kovalenzbindung oder $-CH_2CH_2-$bezeichnen, wobei zwei aromatische Ringe immer durch eine einfache Kovalenzbindung verknüpft sind; $R^{16}$ Wasserstoff, Fluor oder Chlor bedeutet; $R^{17}$ Cyano, Fluor oder Chlor darstellt; $R^{18}$ Wasserstoff oder Fluor bezeichnet; $R^{19}$ Fluor oder Chlor darstellt.

$R^{10}$, $R^{11}$, $R^{12}$, $R^{13}$, $R^{14}$ und $R^{15}$ besitzen vorzugsweise höchstens je 12 Kohlenstoffatome, besonders bevorzugt höchstens je 7 Kohlenstoffatome. Bevorzugte Alkenylgruppen sind 1E-Alkenyl, 3E-Alkenyl und 4Z-Alkenyl. Bevorzugte Alkenyloxygruppen sind 2E-Alkenyloxy und 3Z-Alkenyloxy.

Die Erfindung wird durch die folgenden Beispiele weiter veranschaulicht. Im Zusammenhang mit Flüssigkristallphasen und Phasenübergängen bedeuten C eine kristalline Phase, $S_B$ eine smektische B Phase, N eine nematische Phase, N* eine cholesterische Phase und I die isotrope Phase. Die Helixganghöhe wird mit p bezeichnet und die Wellenlänge des selektiv reflektierten, zirkular polarisierten Lichts mit $\lambda_{max}$. Optische Antipoden besitzen jeweils "spiegelbildliche Eigenschaften", d.h. gleiche Schmelzpunkte etc., führen aber zu entgegengesetztem Helixdrehsinn und entgegengesetzter Zirkularpolarisation des reflektierten Lichts.

Beispiel 1

Eine Suspension von 1,05 g 4-(trans-4-Heptylcyclohexyl)-N-[(S)-2-hydroxy-1-isopropyläthyl)benzamid in 40 ml Acetonitril wurde tropfenweise bei 0°C und unter Stickstoffbegasung mit 0,7 ml Thionylchlorid versetzt. Das Reaktionsgemisch wurde über Nacht bei 0°C gerührt, auf 100 ml gesättigte Kaliumcarbonatlösung gegossen und dann dreimal mit je 50 ml Diäthyläther extrahiert. Die vereinigten organischen Phasen wurden zweimal mit je 50 ml gesättigter Kochsalz-Lösung gewaschen, über Magnesiumsulfat getrocknet, filtriert und anschliessend eingeengt. Chromatographie des Rückstandes an Kieselgel mit Hexan/Aethylacetat (Vol. 2:1) und Umkristallisation aus Aceton bei -25°C ergab reinen (S)-2-[4-(trans-4-Heptylcyclohexyl)-phenyl]-4-isopropyl-2-oxazolin mit Smp. (C-I) 51°C und Klp. (S-I) 46°C.

Das als Ausgangsmaterial verwendete 4-(trans-4-Heptylcyclohexyl)-N-[(S)-2-hydroxy-1-isopropyläthyl)benzamid wurde wie folgt hergestellt:

2,1 g 4-(trans-4-Heptylcyclohexyl)benzoesäure wurden mit 10 ml Thionylchlorid in Toluol 1 Stunde auf 80°C erhitzt. Die erhaltene Lösung wurde unter vermindertem Druck eingedampft, der Rückstand mit 20 ml absolutem Toluol versetzt und die Lösung nochmals unter vermindertem Druck eingedampft. Das erhaltene Säurechlorid wurde in 50 ml Dichlormethan aufgenommen und einer Lösung von 1,45 g S(+)-2-Amino-3-methyl-1-butanol in 20 ml Dichlormethan bei 0°C und unter Stickstoffbegasung zugetropft. Das Reaktionsgemisch wurde über Nacht bei Raumtemperatur gerührt, dann auf 100 ml Kaliumcarbonat gegossen, während 5 Minuten intensiv gerührt, dann dreimal mit je 50 ml Diäthyläther extrahiert. Die vereinigten organischen Phasen wurden zweimal mit je 100 ml gesättigte Kochsalz-Lösung gewaschen, über Magnesiumsulfat getrocknet, filtriert und anschliessend eingeengt. Dies ergab 1,05 g 4-(trans-4-Heptylcyclohexyl)-N-[(S)-2-hydroxy-1-isopropyläthyl)benzamid.

In analoger Weise können folgende Verbindung hergestellt werden:

(S)-2-[4-(trans-4-Propylcyclohexyl)phenyl]-4-isopropyl-2-oxazolin;
(S)-2-[4-(trans-4-Pentylcyclohexyl)phenyl]-4-isopropyl-2-oxazolin:
(S)-2-[4-(trans-4-Propylcyclohexyl)phenyl]-4-tert.butyl-2-oxazolin;
(S)-2-[4-(trans-4-Pentylcyclohexyl)phenyl]-4-tert.butyl-2-oxazolin;
(S)-2-[4-(trans-4-Heptylcyclohexyl)phenyl]-4-tert.butyl-2-oxazolin;
(S)-2-[4-(trans-4-Vinylcyclohexyl)phenyl]-4-isopropyl-2-oxazolin;
(S)-2-[4-(trans-4-Allylcyclohexyl)phenyl]-4-isopropyl-2-oxazolin;
(S)-2-[4-(trans-4-(3-Butenyl)cyclohexyl)phenyl]-4-isopropyl-2-oxazolin;
(S)-2-[4-(trans-4-(1-Pentenyl)cyclohexyl)phenyl]-4-isopropyl-2-oxazolin;
(S)-2-[4-(trans-4-(3-Pentenyl)cyclohexyl)phenyl]-4-isopropyl-2-oxazolin;
(S)-2-[4-(trans-4-(4-Pentenyl)cyclohexyl)phenyl]-4-isopropyl-2-oxazolin;
(S)-2-[4-(5-Pentyl-2-pyrimidinyl)phenyl]-4-isopropyl-2-oxazolin, Smp. 113°C;
(S)-2-[4-(trans-4-Propylcyclohexyl)-4'-biphenyl]-4-isopropyl-2-oxazolin;
(S)-2-[4-(trans-4-Pentylcyclohexyl)-4'-biphenyl]-4-isopropyl-2-oxazolin, Smp. (C-$S_B$) 132°C, $S_B$-$S_A$ 175°C, Klp. ($S_A$-I) 210°C;
(S)-2-[4-(trans-4-Heptylcyclohexyl)-4'-biphenyl]-4-isopropyl-2-oxazolin;
(S)-2-[4-(trans-4-Propylcyclohexyl)-4'-biphenyl]-4-tert,butyl-2-oxazolin;
(S)-2-[4-(trans-4-Pentylcyclohexyl)-4'-biphenyl]-4-tert-butyl-2-oxazolin;
(S)-2-[4-(trans-4-Hepylcyclohexyl)-4'-biphenyl]-4-tert-butyl-2-oxazolin;
(S)-2-(4-[1-(trans-4-Pentylcyclohexyl(-2-äthyl]phenyl)-2-isopropyl-2-oxazolin;
(S)-2-(4-[(trans-4-Pentylcyclohexyl)methoxy]phenyl)-2-isopropyl-2-oxazolin;
(S)-2-(4-[3-(trans-4-Pentylcyclohexyl)-1-propyloxy]phenyl)-2-isopropyl-2-oxazolin;
(S)-2-(4-[(E)-3-(trans-4-Pentylcyclohexyl)allyloxy]phenyl)-2-isopropyl-2-oxazolin, Smp. 62°C;
(S)-2-(4-[4-(trans-4-Pentylcyclohexyl)-1-butyl]phenyl)-2-isopropyl-2-oxazolin;
(S)-2-(4-[4(E)-(trans-4-Pentylcyclohexyl)-3-butenyl]phenyl)-2-isopropyl-2-oxazolin;
2,2'-(2,6-Naphthyl)-bis-(S)-isopropyl-2-oxazolin, Smp. 182°C;
2,2'-(2,6-Naphthyl)-bis-(S)-tert.butyl-2-oxazolin;
2,2'-(4,4'-Biphenyl)-bis-(S)-isopropyl-2-oxazolin;
2,2'-(4,4'-Biphenyl)-bis-(S)-tert-butyl-2-oxazolin;
(S)-2-(4-Fluorphenyl)-4-isopropyl-2-oxazolin;
(S)-2-(4-Chlorphenyl)-4-isopropyl-2-oxazolin;
(S)-2-(4-Bromphenyl)-4-isopropyl-2-oxazolin;
(S)-2-(4-Cyanophenyl)-4-isopropyl-2-oxazolin;
(S)-2-(4-Methylphenyl)-4-isopropyl-2-oxazolin;
(S)-2-(4-Aethylphenyl)-4-isopropyl-2-oxazolin;
(S)-2-(4-Propylphenyl)-4-isopropyl-2-oxazolin, liquid (colourless);

(S)-2-(4-Butylphenyl)-4-isopropyl-2-oxazolin;
(S)-2-(4-Pentylphenyl-4-isopropyl-2-oxazolin;
(S)-2-(4-Hexylphenyl)-4-isopropyl-2-oxazolin;
(S)-2-(4-Heptylphenyl)-4-isopropyl-2-oxazolin;
(S)-2-(4-Octylphenyl)-4-isopropyl-2-oxazolin;
(S)-2-(4-Methoxyphenyl)-4-isopropyl-2-oxazolin;
(S)-2-(4-Aethoxyphenyl)-4-isopropyl-2-oxazolin;
(S)-2-(4-Propyloxyphenyl)-4-isopropyl-2-oxazolin;
(S)-2-(4-Butyloxyphenyl)-4-isopropyl-2-oxazolin;
(S)-2-(4-Pentyloxyphenyl)-4-isopropyl-2-oxazolin;
(S)-2-(4-Hexyloxyphenyl)-4-isopropyl-2-oxazolin;
(S)-2-(4-Heptyloxyphenyl)-4-isopropyl-2-oxazolin, Smp. 31°C;
(S)-2-(4-Fluor-4'-biphenyl)-4-isopropyl-2-oxazolin;
(S)-2-(4-Chlor-4'-biphenyl)-4-isopropyl-2-oxazolin;
(S)-2-(4-Brom-4'-biphenyl)-4-isopropyl-2-oxazolin;
(S)-2-(4-Cyano-4'-biphenyl)-4-isopropyl-2-oxazolin;
(S)-2-(4-Methyl-4'-biphenyl)-4-isopropyl-2-oxazolin;
(S)-2-(4-Aethyl-4'-biphenyl)-4-isopropyl-2-oxazolin;
(S)-2-(4-Propyl-4'-biphenyl)-4-isopropyl-2-oxazolin;
(S)-2-(4-Butyl-4'-biphenyl)-4-isopropyl-2-oxazolin;
(S)-2-(4-Pentyl-4'-biphenyl)-4-isopropyl-2-oxazolin;
(S)-2-(4-Methoxy-4'-biphenyl)-4-isopropyl-2-oxazolin;
(S)-2-(4-Aethoxy-4'-biphenyl)-4-isopropyl-2-oxazolin;
(S)-2-(4-Propyloxy-4'-biphenyl)-4-isopropyl-2-oxazolin;
(S)-2-(4-Butyloxy-4'-biphenyl)-4-isopropyl-2-oxazolin;
(S)-2-(4-Pentyloxy-4'-biphenyl)-4-isopropyl-2-oxazolin, Smp. 125°C;
(S)-2-(4-Heptyloxy-4'-biphenyl)-4-isopropyl-2-oxazolin.
(S)-2-[trans-4-(trans-4-Pentylcyclohexyl)cyclohexyl]-4-isopropyl-2-oxazolin, Smp. (C-$S_B$) 38°C, Klp. ($S_B$-N) 126°C;

<u>Beispiel 2</u>

2,0 g (S)-2-(4-Carboxyphenyl)-4-isopropyl-2-oxazolin, 0,5 g (S)-Milchsäuremethylester und 0,1 g 4-(Dimethylamino)pyridin wurden in 50 ml Dichlormethan gelöst und die Lösung innert 10 Minuten unter Rühren portionenweise mit 1,4 g N,N'-Dicyclohexylcarbodiimid versetzt. Das Gemisch wurde über Nacht bei Raumtemperatur gerührt und dann filtriert. Das Filtrat wurde mit Dichlormethan verdünnt, zweimal mit je 50 ml gesättigter Natriumcarbonatlösung und dann mit Wasser gewaschen, über Magnesiumsulfat getrocknet und eingeengt. Das erhaltene Rohprodukt wurde an Kieselgel mit Toluol chromatographisch gereinigt. Der erhaltene (S)-2-[4-([(S)-1-(Methoxycarbonyl)-1-äthoxy]carbonyl)phenyl]-4-isopropyl-2-oxazolin wurde aus Aethanol umkristallisiert;

Der als Ausgangsmaterial verwendete (S)-2-(4-Carboxyphenyl) -4-isopropyl-2-oxazolin wurde wie folgt hergestellt:

Ein Gemisch von 3 g (S)-2-(4-[Methoxycarbonyl]phenyl)-4-isopropyl-2-oxazolin, 3 g Kaliumhydroxid, 5 ml Wasser und 50 ml Methanol wurde 4 Stunden auf dem Oelbad auf 75°C erhitzt. Die abgekühlte Mischung wurde mit 10 ml eisgekühlter 3N Salzsäure versetzt und die freigesetzte Säure in 150 ml Diäthyläther aufgenommen. Die abgetrennte wässrige Phase wurde zweimal mit je 100 ml Diäthyläther nachextrahiert. Die vereinigten organischen Phasen wurden mit 50 ml 2N Natriumcarbonat-Lösung und mehrmals mit Wasser gewaschen, über Magnesiumsulfat getrocknet und eingeengt. Umkristallisation des erhaltenen Rohproduktes aus Aethanol ergab 2,0 g (S)-2-(4-Carboxyphenyl)-4-isopropyl-2-oxazolin.

In analoger Weise können folgende Verbindungen hergestellt werden:

(S)-2-[4-([(S)-1-(Aethoxycarbonyl)-1-äthoxy]carbonyl)-phenyl]-4-isopropyl-2-oxazolin:
(S)-2-[4-([(S)-1-(Propyloxycarbonyl)-1-äthoxy]carbonyl)phenyl]-4-isopropyl-2-oxazolin;
(S)-2-[4-([(S)-1-(Butyloxycarbonyl)-1-äthoxy]carbonyl)phenyl]-4-isopropyl-2-oxazolin;
(S)-2-[4-([(S)-2-Octyl]carbonyl)phenyl]-4-isopropyl-2-oxazolin und die optischen Antipoden der genannten Verbindungen.

<u>Beispiel 3</u>

Zur Messung der induzierten Ganghöhe (Pitch) und deren Temperaturabhängigkeit in Flüssigkristallmaterialien

wurde die folgende Flüssigkristall-Grundmischung BM-1 verwendet:

| | |
|---|---|
| 5,36 Gew.% | 4'-Aethyl-4-cyanobiphenyl, |
| 3,18 " | 4'-Propyl-4-cyanobiphenyl, |
| 6,08 " | 4'-Butyl-4-cyanobiphenyl, |
| 6,53 " | 4-(trans-4-Propylcyclohexyl)benzonitril, |
| 14,67 " | 4-(trans-4-Pentylcyclohexyl)benzonitril, |
| 5,21 " | 4-Aethyl-1-(trans-4-propylcyclohexyl)benzol, |
| 16,54 " | 4-Aethoxy-1-[2-(trans-4-propylcyclohexyl)äthyl]benzol, |
| 5,60" | 4 "-Pentyl-4-cyano-p-terphenyl, |
| 5,71 " | 4'-(trans-4-Pentylcyclohexyl)-4-cyanobiphenyl, |
| 15,95 " | 1-[2-(trans-4-Butylcyclohexyl)äthyl]-4-(trans-4-pentylcyclohexyl)benzol, |
| 4,74 " | 4-[2-(trans-4-Butylcyclohexyl)äthyl]-4'-(trans-4-pentylcyclohexyl)biphenyl, |
| 7,59 " | 4-[2-(trans-4-Butylcyclohexyl)äthyl]-4'-(trans-4-pentylcyclohexyl)-1,1'-äthylendibenzol, |
| 2,84 " | trans-4-[2-(trans-4-Propylcyclohexyl)äthyl]-cyclohexancarbonsäure-4-cyanophenylester; |
| Smp. <-30°C, Klp. (N-I) 90°C; $\Delta\varepsilon$ = 8,5, $\Delta n$ = 0,139 und $\eta$ = 22 mPa·s gemessen bei 22°C. | |

Flüssigkristall-Grundmischung BM-1 wurde jeweils mit einem der folgenden optisch aktiven Dotierstoffe versetzt:

D-1 = (S)-2-[4-(trans-4-Heptylcyclohexyl)phenyl]-4-isopropyl-2-oxazolin,
D-2 = 2,2'-(2,6-Naphthyl)-bis-(S)-isopropyl-2-oxazolin.

Für die chiral dotierten Gemische wurden die in Tabelle 1 zusammengestellten Ergebnisse erhalten, wobei A, B und C die Parameter der entwicklung

$$\frac{1}{pc} = A + BT_1 + CT_1^2$$

bezeichnen und p, c und $T_1$ die folgenden Bedeutungen haben:

$T_1$ = T-22°C
T = Temperatur in °C
p = Ganghöhe in µm (ein positiver Wert bedeutet rechtsdrehende Helixstruktur und ein negativer Wert bedeutet linksdrehende Helixstruktur)
c = Konzentration des optisch aktiven Dotierstoffes in Gew.-%.

Tabelle 1

| Mischung | Dotierstoff | A [$10^{-2} \cdot \mu m^{-1} \cdot$ Gew.$\%^{-1}$] | B [$10^{-4} \cdot \mu m^{-1} \cdot$ Gew.$\%^{-1} \cdot °C^{-1}$] | C [$10^{-6} \cdot \mu m^{-1} \cdot$ Gew.$\%^{-1} \cdot °C^{-2}$] | p•c (bei 22°C ) [µm•Gew.%] |
|---|---|---|---|---|---|
| M-1 | 1,0 Gew.% D-1 | -5,36 | 0,805 | 0,800 | -18,65 |
| M-2 | 1,0 Gew.% D-2 | +5,75 | 1,234 | 0,656 | +17,39 |

Beispiel 4

Eine Lösung von 0,8 g (S)-2-(4-Formylphenyl)-4-isopropyl-2-oxazolin und 1 g L(+)-Weinsäuredimethylester in 50 ml Toluol wurde mit 0,1 g Toluol-4-sulfonsäure versetzt. Das Gemisch wurde 2,5 Stunden zum Sieden erhitzt, wobei das entstandene Wasser gleichzeitig abdestilliert wurde. Dann wurden 4 Tropfen Triäthylamin zum Reaktionsgemisch zugegeben. Nach dem Erkalten wurde das Gemisch mit 20 ml 1N Natriumhydrogencarbonat-Losung und zweimal mit je 20 ml Wasser gewaschen, über Natriumsulfat getrocknet, filtriert und eingeengt. Chromatographie des Rückstandes an Kieselgel mit Toluoin/Aethylacetat (Vol. 1:1) und Umkristallisation aus Aethanol ergab reinen (4R,5R)-2-(4-[(S)-4-isopropyl-2-oxazolinyl]phenyl)-1,3-dioxolan-4,5-dicarbonsäure-dimethylester;

Das als Ausgangsmaterial verwendete (S)-2-(4-Formylphenyl)-4-isopropyl-2-oxazolin kann wie folgt hergestellt werden:

Eine Lösung von 2 g (S)-2-(4-Bromphenyl)-4-isopropyl-2-oxazolin in 20 ml absolutem Tetrahydrofuran wurde tropfenweise bei -78°C und unter Stickstoffbegasung mit 5 ml 1,6M Butyllithium in Hexan versetzt, 2 Stunden bei dieser Temperatur gerührt, dann tropfenweise mit 2 ml absolutem N,N-Dimethylformamid versetzt und dann langsam auf Raumtemperatur aufwärmt. Das Reaktionsgemisch wurde auf 100 ml Wasser gegossen und dreimal mit je 50 ml Diäthyläther extrahiert. Die vereinigten organischen Phasen wurden zweimal mit je 100 ml gesättigter Kochsalz-Lösung gewaschen, über Magnesiumsulfat getrocknet, filtriert und dann eingeengt. Chromatographie des Rückstandes an Kieselgel mit Hexan/Aethylacetat (Vol. 9:1) ergab 0,8 g reines (S)-2-(4-Formylphenyl)-4-isopropyl-2-oxazolin.

In analoger Weise können folgende Verbindungen hergestellt werden:

(4R,5R)-2-(4-[(S)-4-isopropyl-2-oxazolinyl]phenyl)-1,3-dioxolan-4,5-dicarbonsäure-diethylester;
(4R,5R)-2-(4-[(S)-4-isopropyl-2-oxazolinyl]phenyl)-1,3-dioxolan-4,5-dicarbonsäure-diisopropylester;
(4R,5R)-2-(4-[(S)-4-isopropyl-2-oxazolinyl]phenyl)-1,3-dioxolan-4,5-dicarbonsäure-dibutylester;
(4R,5R)-2-(4-[(S)-4-tert.butyl-2-oxazolinyl]phenyl)-1,3-dioxolan-4,5-dicarbonsäure-dimethylester;
(4R,5R)-2-(4-[(S)-4-tert.butyl-2-oxazolinyl]phenyl)-1,3-dioxolan-4,5-dicarbonsäure-diäthylester;
(4R,5R)-2-(4-[(S)-4-tert.butyl-2-oxazolinyl]phenyl)-1,3-dioxolan-4,5-dicarbonsäure-diisopropylester;
(4R,5R)-2-(4-[(S)-4-tert.butyl-2-oxazolinyl]phenyl)-1,3-dioxolan-4,5-dicarbonsäure-dibutylester;

Beispiel 5

1,0 g (R)-2-(4-Formylphenyl)-4-isopropyl-2-oxazolin, 1,0 g (2S,3R)-2-Octyl-1,3-butandiol, 0,1 g p-Toluol-sulfonsäure in 50 ml absolutem Toluoi wurden in analoger Weise zu Beispiel 4 zu 2-(4-[2S,4R,5S]-4-Methyl-5-octyl-m-dioxan-2-yl]phenyl)-(R)-4-isopropyl-2-oxazolin umgesetzt.

In analoger Weise können folgende Verbindungen hergestellt werden:

2(4-[2S,4R,5S]-4-Methyl-5-methyl-m-dioxan-2-yl]phenyl)-(R)-4-isopropyl-2-oxazolin;
2-(4-[2S,4R,5S]-4-Methyl-5-äthyl-m-dioxan-2-yl]phenyl)-(R)-4-isopropyl-2-oxazolin;
2-(4-[2S,4R,5S]-4-Methyl-5-propyl-m-dioxan-2-yl]phenyl)-(R)-4-isopropyl-2-oxazolin;
2-(4-[2S,4R,5S]-4-Methyl-5-butyl-m-dioxan-2-yl]phenyl)-(R)-4-isopropyl2-oxazolin;
2-(4-[2S,4R,5S]-4-Methyl-5-pentyl-m-dioxan-2-yl]phenyl)-(R)-4-isopropyl-2-oxazolin;
2-(4-[2S,4R,5S]-4-Methyl-5-hexyl-m-dioxan-2-yl]phenyl)-(R)-4-isopropyl-2-oxazolin;
2-(4-[2S,4R,5S]-4-Methyl-5-heptyl-m-dioxan-2-yl]phenyl)-(R)-4-isopropyl-2-oxazolin;
2-(4-[2S,4R,5S]-4-Methyl-5-vinyl-m-dioxan-2-yl]phenyl)-(R)-4-isopropyl-2-oxazolin;
2-(4-[2S,4R,5S]-4-Methyl-5-allyl-m-dioxan-2-yl]phenyl)-(R)-4-isopropyl-2-oxazolin;
2-(4-[2S,4R,5S]-4-Methyl-5-(3-butenyl)-m-dioxan-2-yl]phenyl)-(R)-4-isopropyl-2-oxazolin;
2-(4-[2S,4R,5S]-4-Methyl-5-(4-pentenyl)-m-dioxan-2-yl]phenyl)-(R)-4-isopropyl-2-oxazolin;
2-(4-[2S,4R,5S]-4-Methyl-5-methyl-m-dioxan-2-yl]phenyl)-(R)-4-tert.butyl-2-oxazolin;
2-(4-[2S,4R,5S]-4-Methyl-5-äthyl-m-dioxan-2-yl]phenyl)-(R)-4-tert.butyl-2-oxazolin;
2-(4-[2S,4R,5S]-4-Methyl-5-propyl-m-dioxan-2-yl]phenyl)-(R)-4-tert.butyl-2-oxazolin;
2-(4-[2S,4R,5S]-4-Methyl-5-butyl-m-dioxan-2-yl]phenyl)-(R)-4-tert.butyl-2-oxazolin;
2-(4-[2S,4R,5S]-4-Methyl-5-pentyl-m-dioxan-2-yl]phenyl)-(R)-4-tert.butyl-2-oxazolin;
2-(4-[2S,4R,5S]-4-Methyl-5-hexyl-m-dioxan-2-yl]phenyl)-(R)-4-tert.butyl-2-oxazolin;
2-(4-[2S,4R,5S]-4-Methyl-5-heptyl-m-dioxan-2-yl]phenyl)-(R)-4-tert.butyl-2-oxazolin;
2-(4-[2S,4R,5S)-4-Methyl-5-octyl-m-dioxan-2-yl]phenyl)-(R)-4-tert.butyl-2-oxazolin;
2-(4-[2S,4R,5S]-4-Methyl-5-vinyl-m-dioxan-2-yl]phenyl)-(R)-4-tert.butyl-2-oxazolin;
2-(4-[2S,4R,5S]-4-Methyl-5-allyl-m-dioxan-2-yl]phenyl)-(R)-4-tert.butyl-2-oxazolin;
2-(4-[2S,4R,5S]-4-Methyl-5-(3-butenyl)-m-dioxan-2-yl]phenyl)-(R)-4-tert.butyl-2-oxazolin;
2-(4-[2S,4R,5S]-4-Methyl-5-(4-pentenyl)-m-dioxan-2-yl]phenyl)-(R)-4-tert.butyl-2-oxazolin.

**Patentansprüche**

1. Optisch aktive Verbindungen der allgemeinen Formel

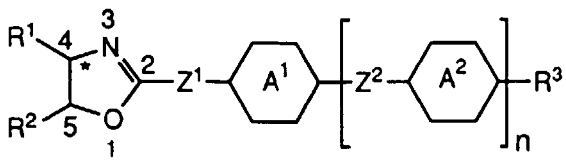

I

worin n für die Zahl 0 oder 1 steht; $R^3$ eine Gruppe $R^4$ oder eine Gruppe der allgemeinen Formeln

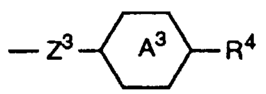

II

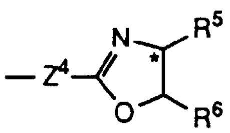

III

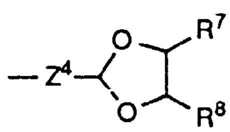

IV

oder

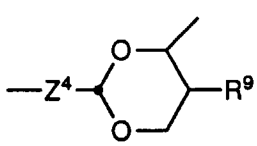

V

bezeichnet; $A^1$, $A^2$ und $A^3$ unabhängig voneinander unsubstituiertes oder einfach oder mehrfach mit Halogen, Cyano und/oder Methyl substituiertes 1,4-Phenylen, in welchem - falls unsubstituiert - gegebenenfalls 1 oder 2 CH-Gruppen durch Stickstoff ersetzt sind, trans- trans-1,4-Cyclohexylen, trans- 1,3-Dioxan-2,5-diyl, Bicyclo[2.2.2.]octan-1,4-diyl, Naphthalin-2,6-diyl, Tetralin-2,6-diyl oder trans-Decalin-2,6-diyl darstellen; $Z^1$ und $Z^4$ unabhängig voneinander eine einfache Kovalenzbindung oder $-CH_2CH_2-$ bedeuten; $Z^2$ und $Z^3$ unabhängig voneinander eine einfache Kovalenzbindung, $-CH_2CH_2-$, -COO-, -OOC-, $-CH_2O-$, $-OCH_2-$, $-C{\equiv}C-$, $-(CH_2)_4-$, $-(CH_2)_3O-$, $-O(CH_2)_3-$ oder die trans-Form von $-CH{=}CH-CH_2CH_2-$, $-CH_2CH_2-CH{=}CH-$, $-CH{=}CH-CH_2O-$ oder $-OCH_2-CH{=}CH-$ bezeichnen; $R^1$ und $R^5$ unabhängig voneinander Alkyl mit 1 bis 12 Kohlenstoffatomen, Alkoxymethyl mit 2 bis 12 Kohlenstoffatomen, p-Alkoxyphenyl oder p-Alkoxybenzyl bedeuten; $R^2$ und $R^6$ unabhängig voneinander Wasserstoff, Alkyl mit 1 bis 12 Kohlenstoffatomen oder Phenyl bedeuten; $R^7$ und $R^8$ unabhängig voneinander Wasserstoff, Alkyl mit 1 bis 12 Kohlenstoffatomen, Phenyl oder Alkoxycarbonyl mit 2 bis 12 Kohlenstoffatomen bedeuten, mit der Massgabe, dass nicht $R^7$ und $R^8$ gleichzeitig Wasserstoff darstellen; $R^9$ unsubstituiertes oder einfach oder mehrfach mit Fluor substituiertes Alkyl oder Alkenyl mit 1 bzw. 2 bis 9 Kohlenstoffatomen, in welchen eine $-CH_2-$Gruppe durch -O- ersetzt sein kann, bedeutet; $R^4$ Wasserstoff, Halogen, Cyano, Trifluormethyl, Trifluormethoxy oder unsubstituiertes oder einfach oder mehrfach mit Halogen, Cyano und/oder Methyl substituiertes Alkyl oder Alkenyl mit 1 bzw. 2 bis 12 Kohlenstoffatomen bezeichnet, in welchen eine oder zwei nicht benachbarte Methylengruppen durch -O-, -COO- und/oder -OOC- ersetzt sein können; und der in Formel I dargestellte Oxazolin-Ring in optisch aktiver Form vorliegt, mit der Massgabe, dass nicht gleichzeitig $R^3$ eine Gruppe der Formel III und n 0 bedeuten.

**2.** Optisch aktive Verbindungen gemäss Anspruch 1 der allgemeinen Formel

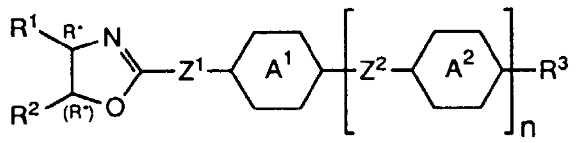

IA

worin n die Zahl 0 oder 1 bedeutet; $R^3$ eine Gruppe $R^4$ oder eine Gruppe der allgemeinen Formel

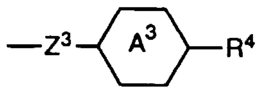

II

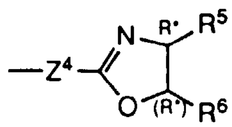

IIIA

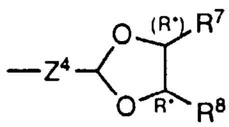

IVA

oder

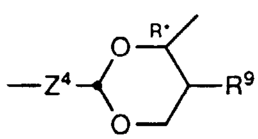

VA

bezeichnet; n, $A^1$, $A^2$, $A^3$, $Z^1$, $Z^2$, $Z^3$, $Z^4$, $R^1$, $R^2$, $R^4$, $R^5$, $R^6$, $R^7$, $R^8$ und $R^9$ die in Anspruch 1 angegebenen Bedeutungen haben; das Chiralitätszentrum (R*) fehlt, wenn $R^2$ Wasserstoff bedeutet, und steht für R*, wenn $R^2$ von Wasserstoff verschieden ist; und die mit R* bezeichneten, asymmetrischen Kohlenstoffatomen alle in der R-Konfiguration oder alle in der S-Konfiguration vorliegen.

**3.** Optisch aktive Verbindungen nach Anspruch 1 oder 2, dadurch gekennzeichnet, dass einer der Ringe $A^1$, $A^2$ und $A^3$ unsubstituiertes oder einfach oder mehrfach mit Halogen substituiertes 1,4-Phenylen oder Pyridin-2,5-diyl, Pyrimidin-2,5-diyl oder trans-1,4-Cyclohexylen darstellt, die andern der Ringe $A^1$, $A^2$ und $A^3$ unabhängig voneinander unsubstituiertes oder einfach oder mehrfach mit Halogen substituiertes 1,4-Phenylen oder trans-1,4-Cyclohexylen darstellen, eine der Gruppen $Z^2$ und $Z^3$ eine einfache Kovalenzbindung, $-CH_2CH_2-$, -COO- oder -OOC- bezeichnet, und die andere der Gruppen $Z^2$ und $Z^3$ eine einfache Kovalenzbindung bezeichnet.

**4.** Optisch aktive Verbindungen nach Anspruch 3, dadurch gekennzeichnet, dass $A^1$, $A^2$ und $A^3$ unabhängig voneinander unsubstituiertes oder einfach oder mehrfach mit Halogen substituiertes 1,4-Phenylen oder trans-1,4-Cyclohexylen darstellen oder eine der Gruppen $A^1$ , $A^2$ und $A^3$ auch Pyrimidin-2,5-diyl darstellt, eine der Gruppen $Z^2$ und $Z^3$ eine einfache Kovalenzbindung oder $-CH_2CH_2-$ bezeichnet, und die andere der Gruppen $Z^2$ und $Z^3$ eine einfache Kovalenzbindung bezeichnet.

5. Optisch aktive Verbindungen nach einem der Ansprüche 1 bis 4, gekennzeichnet durch die allgemeinen Formeln

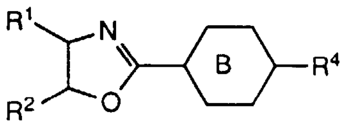

I-11

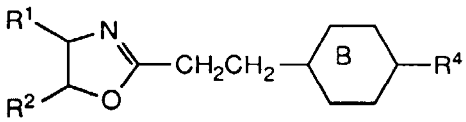

I-12

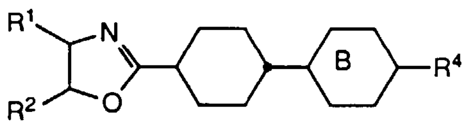

I-13

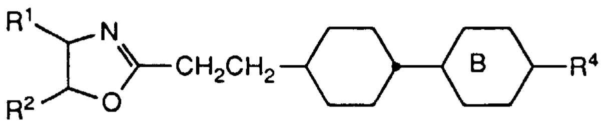

I-14

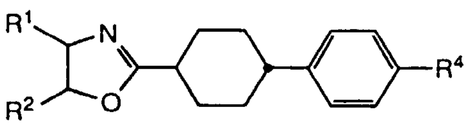

I-15

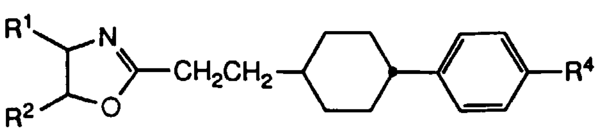

I-16

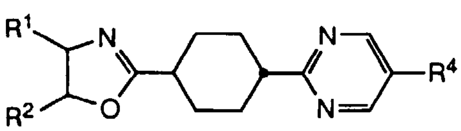

I-17

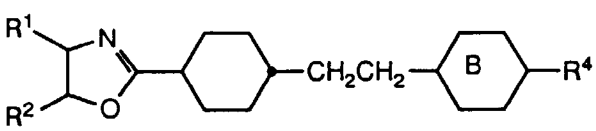

I-18

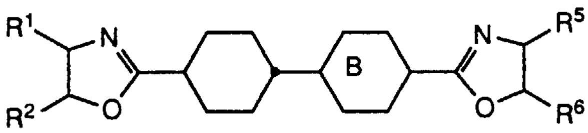

I-21

worin $R^1$, $R^2$, $R^4$, $R^5$, $R^6$, $R^7$, $R^8$ und $R^9$ die in Anspruch 1 gegebenen Bedeutungen haben; und Ring B unsubstituiertes oder einfach oder mehrfach mit Halogen substituiertes 1,4-Phenylen oder trans-1,4-Cyclohexylen darstellt.

**6.** Optisch aktive Verbindungen nach einem der Ansprüche 1 bis 5, dadurch gekennzeichnet, dass $R^5$ die gleiche Bedeutung und am benachbarten C-Atom die gleiche Konfiguration wie $R^1$ besitzt; $R^6$ die gleiche Bedeutung und am benachbarten C-Atom die gleiche Konfiguration wie $R^2$ besitzt; und $R^1$ und $R^2$ die in Anspruch 1 angegeben Bedeutungen haben

**7.** Optisch aktive Verbindungen nach einem der Ansprüche 1 bis 6, dadurch gekennzeichnet, dass $R^4$ Halogen, Cyano, Trifluormethyl, Trifluormethoxy, unsubstitutiertes Alkyl, Alkenyl, Alkoxy, Alkenyloxy, 1-(Alkoxycarbonyl) äthoxy 1-(Alkoxycarbonyl)äthoxycarbonyl oder mit 1 bzw. 2 bis 7 C-Atomen, vorzugsweise mit 1 bzw. 2 bis 5 C-Atomen bezeichnet.

**8.** Flüssigkristallines Gemisch enthaltend ein flüssigkristallines Trägermaterial und eine oder mehrere optisch aktive Verbindungen gemäss Ansprüchen 1 bis 7.

**9.** Verwendung flüssigkristalliner Gemische gemäss Anspruch 9 für optisch oder elektro-optische Zwecke.

## Claims

**1.** Optically active compounds of the general formula

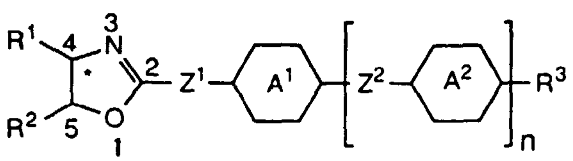

I

wherein n stands for the number 0 or 1; $R^3$ denotes a group $R^4$ or a group of the general formula

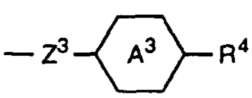

II

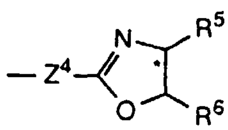

III

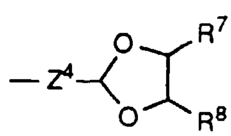

IV

or

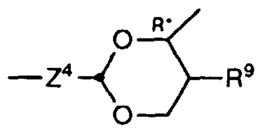

V

$A^1$, $A^2$ and $A^3$ each independently represent 1,4-phenylene, which is unsubstituted or mono- or multiply-substituted with halogen, cyano and/or methyl and in which - where it is unsubstituted - 1 CH group or 2 CH groups is/are optionally replaced by nitrogen, trans-1,4-cyclohexylene, trans-1,3-dioxane-2,5-diyl, bicyclo[2.2.2.]octane-1,4-diyl, naphthalene-2,6-diyl, tetralin-2,6-diyl or trans-decalin-2,6-diyl; $Z^1$ and $Z^4$ each independently signify a single covalent bond or $-CH_2CH_2-$; $Z^2$ and $Z^3$ each independently denote a single covalent bond, $-CH_2CH_2-$, -COO-, -OOC-, $-CH_2O-$, $-OCH_2-$, -C≡C-, $-(CH_2)_4-$, $-(CH_2)_3O-$, $-O(CH_2)_3-$ or the trans form of $-CH=CH-CH_2CH_2-$, $-CH_2CH_2-CH=CH-$, $-CH=CH-CH_2O-$ or $-OCH_2-CH=CH-$; $R^1$ and $R^5$ each independently signify alkyl with 1 to 12 carbon atoms, alkoxymethyl with 2 to 12 carbon atoms, p-alkoxyphenyl or p-alkoxybenzyl; $R^2$ and $R^6$ each independently signify hydrogen, alkyl with 1 to 12 carbon atoms or phenyl; $R^7$ and $R^8$ each independently signify hydrogen, alkyl with 1 to 12 carbon atoms, phenyl or alkoxycarbonyl with 2 to 12 carbon atoms, with the proviso that $R^7$ and $R^8$ do not simultaneously represent hydrogen; $R^9$ signifies alkyl or alkenyl with 1 to 9 and, respectively, 2 to 9 carbon atoms, which is unsubstituted or mono- or multiply-substituted with fluorine and in which one $-CH_2-$ group can be replaced by -O-; $R^4$ denotes hydrogen, halogen, cyano, trifluoromethyl, trifluoromethoxy or alkyl or alkenyl with 1 to 12 and, respectively, 2 to 12 carbon atoms, which is unsubstituted or mono- or multiply-substituted with halogen, cyano and/or methyl and in which one methylene group or two non-adjacent methylene groups can be replaced by -O-, -COO-and/or -OOC-; and the oxazoline ring set forth in formula I is present in optically active form; with the proviso that simultaneously $R^3$ does not signify a group of formula III and n does not signify 0.

**2.** Optically active compounds in accordance with claim 1 of the general formula

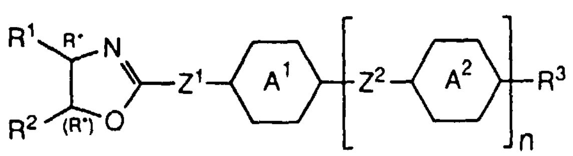

IA

wherein n signifies the number 0 or 1; $R^3$ denotes a group $R^4$ or a group of the general formula

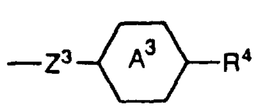

II

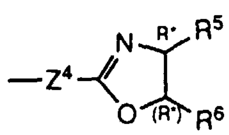

IIIA

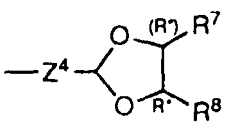

IVA

or

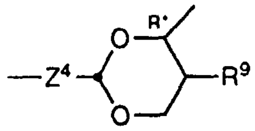

VA

n, $A^1$, $A^2$, $A^3$, $Z^1$, $Z^2$, $Z^3$, $Z^4$, $R^1$, $R^2$, $R^4$, $R^5$, $R^6$, $R^7$, $R^8$ and $R^9$ have the significances given in claim 1; the chiral center (R*) is absent when $R^2$ signifies hydrogen and stands for R* when $R^2$ is different from hydrogen; and the asymmetric carbon atoms denoted by R* are all present in the R-configuration or are all present in the S-configuration.

3. Optically active compounds according to claim 1 or 2, characterized in that one of rings $A^1$, $A^2$ and $A^3$ represents phenylene which is unsubstituted or mono- or multiply-substituted with halogen, or pyridine-2,5-diyl, pyrimidine-2,5-diyl, trans-1,4-cyclohexylene or trans-1,3-dioxane-2,5-diyl, the others of rings $A^1$, $A^2$ and $A^3$ each independently represent 1,4-phenylene which is unsubstituted or mono- or multiply-substituted with halogen, or trans-1,4-cyclohexylene, one of groups $Z^2$ and $Z^3$ denotes a single covalent bond, $-CH_2CH_2-$, -COO- or -OOC- and the other of groups $Z^2$ and $Z^3$ denotes a single covalent bond.

4. Optically active compounds according to claim 3, characterized in that $A^1$, $A^2$ and $A^3$ each independently represent 1,4-phenylene which is unsubstituted or mono- or multiply-substituted with halogen, or trans-1,4-cyclohexylene or one of groups $A^1$, $A^2$ and $A^3$ also represents pyrimidine-2,5-diyl or pyridine, one of groups $Z^2$ and $Z^3$ denotes a single covalent bond or $-CH_2CH_2-$ and the other of groups $Z^2$ and $Z^3$ denotes a single covalent bond.

5. Optically active compounds according to any one of claims 1 to 4, characterized by the general formulae

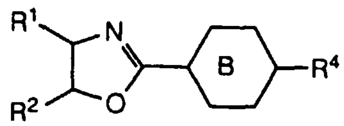

I-11

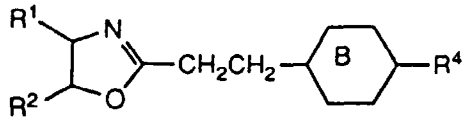

I-12

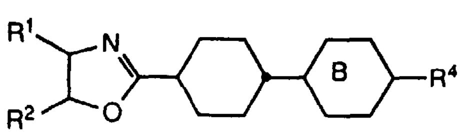

I-13

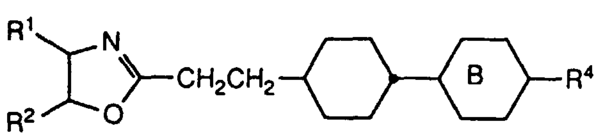

I-14

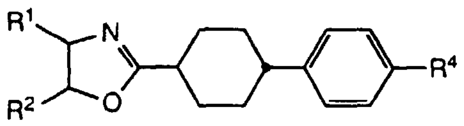

I-15

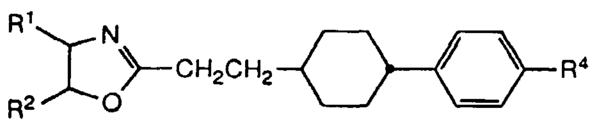

I-16

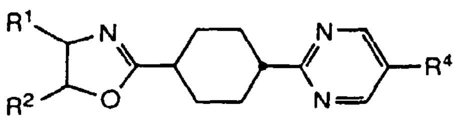

I-17

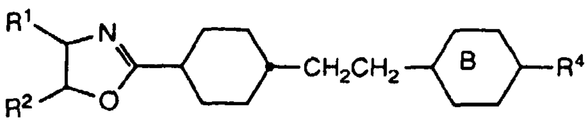

I-18

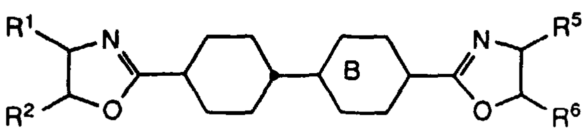

I-21

wherein $R^1$, $R^2$, $R^4$, $R^5$, $R^6$, $R^7$, $R^8$ and $R^9$ have the significances given in claim 1; and ring B represents 1,4phenylene which is unsubstituted or mono- or multiply-substituted with halogen, or trans-1,4-cyclohexylene.

**6.** Optically active compounds according to any one of claims 1 to 5, characterized in that $R^5$ has the same significance and the same configuration at the adjacent carbon atom as $R^1$; $R^6$ has the same significance and the same configuration at the adjacent carbon atom as $R^2$; and $R^1$ and $R^2$ have the significances given in claim 1.

**7.** Optically active compounds according to any one of claims 1 to 6, wherein $R^4$ denotes halogen, cyano, trifluoromethyl, trifluoromethoxy, unsubstituted alkyl, alkenyl, alkoxy, alkenyloxy, 1-(alkoxycarbonyl)ethoxy or 1-(alkoxycarbonyl)ethoxycarbonyl having 1 to 7, and 2 to 7 respectively, carbon atoms, preferably 1 to 5 and 2 to 5, respectively, carbon atoms.

**8.** A liquid crystalline mixture containing a liquid crystalline carrier material and one or more optically active compounds in accordance with claims 1 to 7.

**9.** The use of a liquid crystalline mixture in accordance with claim 9 for optical or electro-optical purposes.

**Revendications**

**1.** Composés optiquement actifs de formule générale

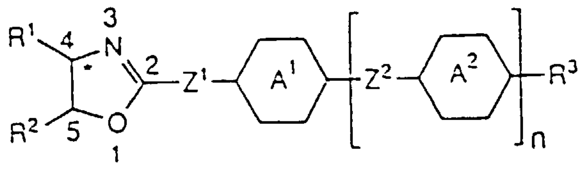

I

où n est égal à 0 ou 1 ; $R^3$ représente un groupe $R^4$ ou un groupe de formules générales

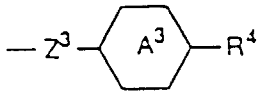

II

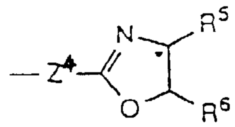

III

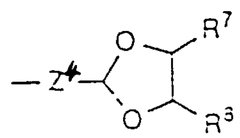

IV

ou

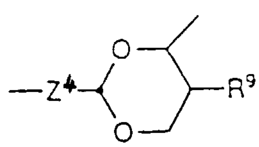

V

$A^1$, $A^2$ et $A^3$ représentent, indépendamment les uns des autres, le 1,4-phénylène non substitué ou mono- ou polysubstitué par un halogène, un cyano et/ou un méthyle et dans lequel, s'il est non substitué, 1 ou 2 groupes CH sont éventuellement remplacés par l'azote, $A^1$, $A^2$ et $A^3$ représentent, indépendamment les uns des autres, le trans-1,4-cyclohexylène, le trans-1,3-dioxane-2,5-diyle, le bicyclo[2.2.2]octane-1,4-diyle, le naphtalène-2,6-diyle, le tétraline-2,6-diyle ou le trans-décaline-2,6-diyle; $Z^1$ et $Z^4$ représentent, indépendamment l'un de l'autre, une liaison covalente simple ou $-CH_2CH_2-$ ; $Z^2$ et $Z^3$ représentent, indépendamment l'un de l'autre,une liaison covalente simple, $-CH_2CH_2-$, -COO-, -OOC-, $-CH_2O-$, $-OCH_2-$, $C{\equiv}C-$, $-(CH_2)_4-$, $-(CH_2)_3O-$, $-O(CH_2)_3-$ ou la forme trans de $-CH{=}CH-CH_2CH_2-$, $-CH_2CH_2-CH{=}CH-$, $-CH{=}CH-CH_2O-$ ou $-OCH_2-CH{=}CH-$ ; $R^1$ et $R^5$ représentent, indépendamment l'un de l'autre, un alkyle comportant 1 à 12 atomes de carbone, un alcoxyméthyle comportant 2 à 12 atomes de carbone, un p-alcoxyphényle ou un p-alcoxybenzyle ; $R^2$ et $R^6$ représentent, indépendamment l'un de l'autre, un hydrogène, un alkyle comportant 1 à 12 atomes de carbone ou un phényle ; $R^7$ et $R^8$ représentent, indépendamment l'un de l'autre, un hydrogène, un alkyle comportant 1 à 12 atomes de carbone, un phényle ou un alcoxycarbonyle comportant 2 à 12 atomes de carbone, sous réserve que $R^7$ et $R^8$ ne représentent pas en même temps un hydrogène ; $R^9$ représente un alkyle ou un alcényle comportant 1 ou 2 à 9 atomes de carbone non substitués ou mono- ou polysubstitués par le fluor, dans lesquels un groupe $CH_2$ peut être remplacé par -O- ; $R^4$ représente un hydrogène, un halogène, un cyano, un trifluorométhyle, un trifluorométhoxy ou un alkyle ou un alcényle comportant 1 ou 2 à 12 atomes de carbone non substitués ou mono- ou polysubstitués par un halogène, un cyano et/ ou un méthyle, dans lesquels un ou deux groupes méthylène non adjacents peuvent être remplacés par -O-, -COO- et/ou -OOC- ; le cycle oxazoline représenté dans la formule I se présentant sous une forme optiquement active, sous réserve qu'en même temps $R^3$ ne représente pas un groupe de formule III et n soit égal à 0.

2. Composés optiquement actifs selon la revendication 1 de formule générale

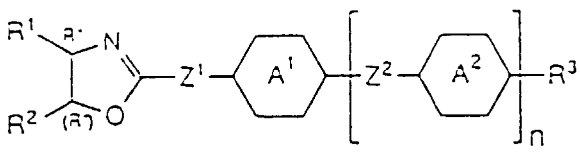

IA

où n est égal à 0 ou 1 ; $R^3$ représente un groupe $R^4$ ou groupe de formules générales

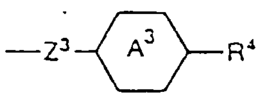

II

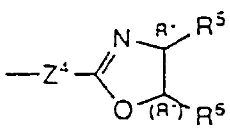

IIIA

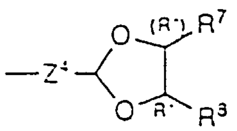

IVA

ou

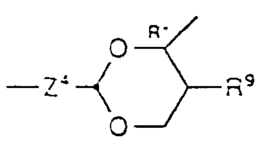

VA

n, $A^1$, $A^2$, $A^3$, $Z^1$, $Z^2$, $Z^3$, $Z^4$, $R^1$, $R^2$, $R^4$, $R^5$, $R^6$, $R^7$, $R^8$ et $R^9$ ayant les significations indiquées dans la revendication 1; le centre de chiralité (R*) manquant lorsque $R^2$ représente un hydrogène et représentant R* lorsque $R^2$ est différent d'un hydrogène ; les atomes de carbone asymétriques désignés par R* se présentant tous dans la configuration R ou tous dans la configuration S.

3. Composés optiquement actifs selon la revendication 1 ou 2, caractérisés en ce que l'un des noyaux $A^1$, $A^2$ et $A^3$ représente le 1,4-phénylène non substitué ou mono- ou polysubstitué par un halogène ou le pyridine-2,5-diyle, le pyrimidine-2,5-diyle ou le trams-1,4-cyclohexylène, les autres des noyaux $A^1$, $A^2$ et $A^3$ représentant, indépendamment l'un de l'autre, le 1,4-phénylène non substitué ou mono- ou polysubstitué par un halogène ou le trans-1,4-cyclohexylène, l'un des groupes $Z^2$ et $Z^3$ représentant une liaison covalente simple, $-CH_2CH_2-$, -COO- ou -OOC-, et l'autre des groupes $Z^2$ et $Z^3$ représentant une liaison covalente simple.

4. Composés optiquement actifs selon la revendication 3, caractérisés en ce que $A^1$, $A^2$ et $A^3$ représentent, indépendamment les uns des autres, le 1,4-phénylène non substitué ou mono- ou polysubstitué par un halogène ou le trans-1,4-cyclohexylène ou l'un des groupes $A^1$, $A^2$ et $A^3$ représentent également le pyrimidine-2,5-diyle, l'un des groupes $Z^2$ et $Z^3$ représente une liaison covalente simple ou $-CH_2CH_2-$ et l'autre des groupes $Z^2$ et $Z^3$ représente une liaison covalente simple.

**5.** Composés optiquement actifs selon l'une des revendications 1 à 4, caractérisés par les formules générales

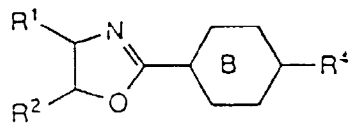

I-11

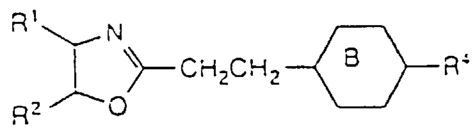

I-12

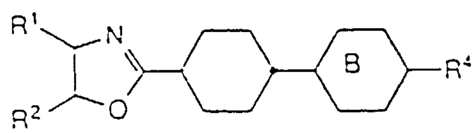

I-13

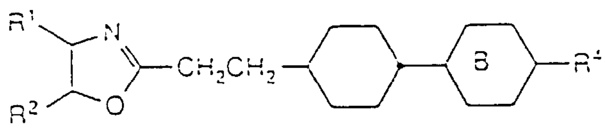

I-14

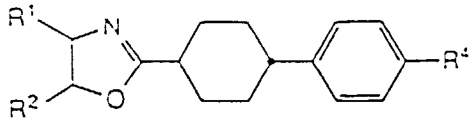

I-15

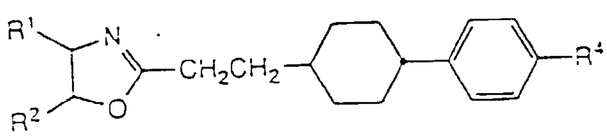

I-16

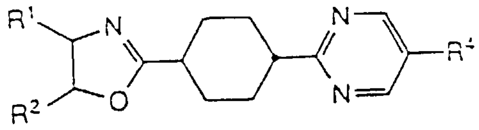

I-17

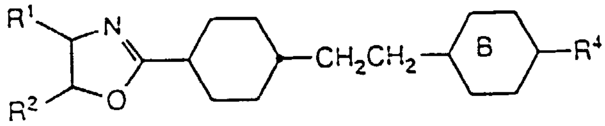

I-18

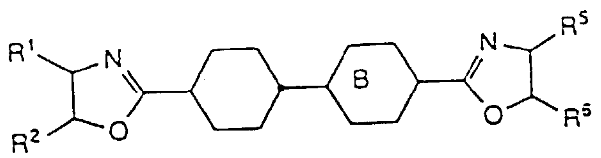

I-21

où $R^1$, $R^2$, $R^4$, $R^5$, $R^6$, $R^7$, $R^8$ et $R^9$ ont les significations données dans la revendication 1 ; et le noyau B représente le 1,4-phénylène non substitué ou mono- ou polysubstitué par un halogène ou le trans-1,4-cyclohexylène.

**6.** Composés optiquement actifs selon l'une des revendications 1 à 5, caractérisés en ce que $R^5$ a la même signification et la même configuration sur l'atome de C adjacent que $R^1$ ; $R^6$ a la même signification et la même configuration sur l'atome de C adjacent que $R^2$, et $R^1$ et $R^2$ ont les significations indiquées dans la revendication 1.

**7.** Composés optiquement actifs selon l'une des revendications 1 à 6, caractérisés en ce que $R^4$ représente un halogène, un cyano, un trifluorométhyle, un trifluorométhoxy, un alkyle, un alcényle, un alcoxy, un alcényloxy, un 1-(alcoxycarbonyl)éthoxy ou un 1-(alcoxycarbonyl)éthoxycarbonyle comportant 1 ou 2 à 7 atomes de C, de préférence 1 ou 2 à 5 atomes de C, non substitués.

**8.** Mélange à cristaux liquides contenant un support à cristaux liquides et un ou plusieurs composés optiquement actifs selon les revendications 1 à 7.

**9.** Utilisation des mélanges à cristaux liquides selon la revendication 9 à des fins optiques ou électro-optiques.